# NEUE EUROPÄISCHE PATENTSCHRIFT

(11) **EP 1 001 027 B2**
(45) Veröffentlichungstag und Bekanntmachung des Hinweises auf die Entscheidung über den Einspruch: **24.06.2009**
(45) Hinweis auf die Patenterteilung: 06.05.2004
(21) Anmeldenummer: 99118670.1
(22) Anmeldetag: 22.09.1999
(51) Int. Cl.: C12N 15/53, C12N 15/67, C12N 15/70, C12N 1/21, C12P 13/02, C12P 7/42

(54) **Verfahren zur Herstellung von Pantothensäure durch Verstärkung von für Ketopantoat-Reduktase kodierende Nukleotidsequenzen**
Process for the reduction of pantothenic acid by amplification of nucleotide sequences coding for ketopantoate-reductase
Procédé pour la préparation d'acide pantothénique par amplification des séquences nucléotides codantes pour la cétopantoate-réductase

(30) Priorität: 09.10.1998 DE 19846499
(43) Veröffentlichungstag der Anmeldung: 17.05.2000
(73) Patentinhaber: Evonik Degussa GmbH, 45128 Essen (DE)
(72) Erfinder: Elischewski, Frank, Dr., 33818 Leopoldshöhe (DE); Kalinowski, Jörn, Dr., 33615 Bielefeld (DE); Pühler, Alfred, Prof. Dr., 33739 Bielefeld (DE); Dusch, Nikole, Dr., 33619 Bielefeld (DE); Dohmen, Jürgen, Dr., 40760 Meerbusch (DE); Farwick, Mike, Dr., 33615 Bielefeld (DE); Thierbach, Georg, Dr., 33613 Bielefeld (DE)

(56) Entgegenhaltungen:
- DATABASE BIOSIS [Online] BIOSCIENCES INFORMATION SERVICE, PHILADELPHIA, PA, US; 1983 PRIMERANO D A ET AL: "ROLE OF ACETO HYDROXY ACID ISOMERO REDUCTASE IN BIOSYNTHESIS OF PANTOTHENIC-ACID IN SALMONELLA-TYPHIMURIUM" Database accession no. PREV198376072046 XP002170048 & JOURNAL OF BACTERIOLOGY, Bd. 153, Nr. 1, 1983, Seiten 259-269, ISSN: 0021-9193
- DATABASE CHEMABS [Online] CHEMICAL ABSTRACTS SERVICE, COLUMBUS, OHIO, US; BAIGORI, MARIO ET AL: "Isolation and characterization of Bacillus subtilis mutants blocked in th synthesis of pantothenic acid" retrieved from STN Database accession no. 115:86365 XP002170049 & J. BACTERIOL. (1991), 173(13), 4240-2 ,
- DATABASE CHEMABS [Online] CHEMICAL ABSTRACTS SERVICE, COLUMBUS, OHIO, US; SHIMIZU, SAKAYU ET AL: "Ketopantoic acid reductase of Pseudomonas maltophilia 845. Purification, characterization, and role in pantothenate biosynthesis" retrieved from STN Database accession no. 109:186033 XP002170050 & J. BIOL. CHEM. (1988), 263(24), 12077-84 ,

## Beschreibung

### Stand der Technik

Die Pantothensäure stellt ein kommerziell bedeutendes Vitamin dar, das in der Kosmetik, der Medizin, der Humanernährung und in der Tierernährung Anwendung findet.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Verbindung. Es wird in einem mehrstufigen Verfahren aus Formaldehyd, Isobutyraldehyd und Cyanid hergestellt. In weiteren Verfahrensschritten wird das racemische Gemisch aufgetrennt und D-Pantolacton mit β-Alanin kondensiert, und man erhält D-Pantothensäure.

Der Vorteil der fermentativen Herstellung durch Mikroorganismen liegt in der direkten Bildung der gewünschten stereoisomeren D-Form.

Verschiedene Arten von Bakterien, wie z. B. Escherichia coli, Arthrobacter ureafaciens, Corynebacterium erythrogenes, Brevibacterium ammoniagenes und auch Hefen, wie z. B. Debaromyces castellii können wie in EPA 0 493 060 gezeigt, in einer Nährlösung, die Glucose, DL-Pantoinsäure und β-Alanin enthält, D-Pantothensäure produzieren. EPA 0 493 060 zeigt weiterhin, daß bei Escherichia coli durch Amplifikation von Pantothensäure-Biosynthesegenen, die auf den Plasmiden pFV3 und pFV5 enthalten sind, in einer Nährlösung, die Glucose, DL-Pantoinsäure und β-Alanin enthält, die Bildung von D-Pantothensäure verbessert wird.

EPA 0 590 857 und US-Patent 5,518,906 beschreiben von Escherichia coli Stamm IFO3547 abgeleitete Mutanten wie FV5714, FV525, FV814, FV521, FV221, FV6051 und FV5069 die Resistenzen gegen verschiedene Antimetabolite wie Salizylsäure, α-Ketobuttersäure, β-Hydroxyasparaginsäure, O-Methylthreonin und α-Ketoisovaleriansäure tragen und in einer Nährlösung, die Glucose enthält Pantoinsäure und in einer Nährlösung, die Glucose und β-Alanin enthält, D-Pantothensäure produzieren. In EPA 0 590 857 und US-Patent 5,518,906 wird weiterhin gezeigt, daß nach Amplifikation der Pantothensäure-Biosynthesegene, die auf dem Plasmid pFV31 enthalten sind, in den oben genannten Stämmen in einer Nährlösung die Glucose enthält die Produktion von D-Pantoinsäure und in einer Nährlösung die Glucose und **β**-Alanin enthält die Produktion von D-Pantothensäure verbessert wird.

In WO 97/10340 wird darüber hinaus gezeigt, daß in Pantothensäure bildenden Stämmen von Escherichia coli durch Erhöhung der Aktivität des Enzyms Acetohydroxysäure-Synthase II, einem Enzym der Valin Biosynthese, die Pantothensäure-Produktion weiter gesteigert werden kann.

Das Dokument Database Biosis [Online] Biossciences Information Service, Philadelphia, PA, US; 1983 Primerano D A et al.: 'Role of Aceto Hydroxy acid isomero reductase in Biosynthesis of Pantothenic-acid in Salmonella-Tyhimurium' Database accession no. PREV198376072046 XP002170048 & Journal of Bacteiology, Bd. 153, Nr. 1983, Seiten 259-269, ISSN: 0021-9193. In diesem Dokument wird die Rolle der Acetohydroxysäure Isomeroreduktase bei der Umwandlung von Ketopantoat in Pantoat beschrieben.

### Aufgabe der Erfindung

Die Erfinder haben sich zur Aufgabe gestellt, neue Grundlagen für ein verbessertes Verfahren zur Herstellung von Pantothensäure bereitzustellen.

### Beschreibung der Erfindung

Das Vitamin Pantothensäure stellt ein kommerziell bedeutendes Produkt dar, das in der Kosmetik, der Medizin, der Humanernährung und in der Tierernährung Anwendung findet. Es besteht daher ein allgemeines Interesse daran verbesserte Verfahren zur Herstellung von Pantothensäure bereitzustellen. Wenn im folgenden Text D-Pantothensäure oder Pantothensäure oder Pantothenat erwähnt werden, sind damit nicht nur die freie Säure sondern auch die Salze der D-Pantothensäure wie z.B. das Calcium-, Natrium-, Ammonium- oder Kaliumsalz gemeint.

Gegenstand der Erfindung ist ein Verfahren zur Herstellung von D-Pantothensäure durch Fermentation diese Säure produzierender Mikroorganismen, dadurch gekennzeichnet, dass man Mikroorganismen einsetzt, in denen man für die Ketopantoatreduktase kodierende Nukleotidsequenzen panE-Gen und gegebenenfalls zusätzlich das ilvC-Gen überexprimiert.

Bei dem Verfahren führt man folgende Schritte durch:
a) Fermentation von Mikroorganismen, ausgwählt aus der Gruppe Gram-negative Bakterien, Gram-positive Bakterien, Hefen und Pilze, in denen man die für das Enzym Ketopantoatreduktase kodierenden Nukleotidsequenzen (panE-Gen) überexprimiert,
b) Anreicherung der Pantothensäure im Medium oder in den Zellen der Mikroorganismen,
c) Isolieren der Pantothensäure.

Der Begriff "Verstärkung" beschreibt die Erhöhung der intrazellulären Aktivität eines oder mehrerer Enzyme, die durch die entsprechende DNA codiert werden, indem man die Kopienzahl des(der) Gene erhöht, einen starken Promotor verwendet oder ein Gen verwendet, das für ein entsprechendes Enzym mit einer hohen spezifischen Aktivität codiert und gegebenenfalls diese Maßnahmen kombiniert.
Es wurde gefunden, daß bei Überexpression des panE-Gens zusammen mit den Genen panB, panC und panD die Bildung der Pantothensäure weiter verbessert wird. Zur Erzielung der Überexpression kann die Kopienzahl der entsprechenden Gene mittels Plasmidvektoren wie z. B. pBR322 (Sutcliffe, COLD SPRING HARBOR SYMPOSIA ON QUANTITATIVE BIOLOGY 1979, 43: 77-90) oder pUC19 (Viera, Gene 1982 19:259-268) erhöht werden, oder es kann die Promotor- und Regulationsregion, die sich stromaufwärts des Strukturgens befindet, mutiert werden. Ein bekanntes Beispiel hierfür ist die lac-UV5-Mutation des lac-Promotors (Winnacker: Gene und Klone, Eine Einführung in die Gentechnologie (Verlag Chemie, Weinheim, Deutschland, 1990). In gleicher Weise wirken Expressionskassetten, die stromaufwärts des Strukturgens eingebaut werden. Diese Methode ist z. B. bei LaVallie et al. (BIO/TECHNOLOGY 11, 187-193 (1993) und in PCT/US97/13359 angewendet worden.

Alternativ kann weiterhin eine Überexpression der betreffenden Gene durch Veränderung der Medienzusammensetzung und Kulturführung erreicht werden. Ein Beispiel hierfür ist die allseits bekannte Regulation der Expression des lac-Operons durch Glukose und Laktose. Die Erfinder haben darüberhinaus herausgefunden, daß die Überexpression des panE-Gens sich in Stämmen vorteilhaft auswirkt, die Resistenzmutationen gegen Metabolite und Antimetabolite wie z. B. Resistenz gegen L-Valin aufweisen. Weiterhin wurde gefunden, daß die Überexpression des panE-Gens sich in Stämmen vorteilhaft auswirkt, die Defektmutationen in Genen von Stoffwechselwegen, wie z.B. dem avtA- oder ilvE-Gen besitzen, die Vorstufen der Pantothensäure umsetzen oder die Pantothensäurebildung mindern.

Die Mikroorganismen, die Gegenstand der vorliegenden Erfindung sind, können Pantothensäure aus Glucose, Saccharose, Lactose, Fructose, Maltose, Melasse, Stärke, Cellulose oder aus Glycerin und Ethanol herstellen. Dabei handelt es sich um Pilze, Hefen oder insbesondere Gram-positive Bakterien z. B. der Gattung Corynebacterium oder um Gram-negative Bakterien, wie z. B. die der Enterobacteriaceae. Bei der Familie der Enterobacteriaceae ist besonders die Gattung Escherichia mit der Art Escherichia coli zu nennen. Innerhalb der Art Escherichia coli sind die sogenannten K-12 Stämme wie z. B. die Stämme MG1655 oder W3110 (Neidhard et al.: Escherichia coli and Salmonella. Cellular and Molecular Biology (ASM Press, Washington D.C.)) oder der Escherichia coli Wildtypstamm IFO3547 (Institut für Fermentation, Osaka, Japan) und davon abgeleitete Mutanten zu nennen. Bei der Gattung Corynebacterium ist insbesondere die Art Corynebacterium glutamicum zu nennen, die in der Fachwelt für ihre Fähigkeit bekannt ist, Aminosäuren zu bilden. Zu dieser Art gehören Wildtypstämme wie z. B. Corynebacterium glutamicum ATCC13032, Brevibacterium flavum ATCC14067, Corynebacterium melassecola ATCC17965 und andere.

Für die Isolierung des ilvC-Gens und des panE-Gens stellt man sich zunächst eine Mutante beispielsweise von Escherichia coli her, die eine Mutation im ilvC-Gen und panE-Gen trägt.

Die Nukleotidsequenz des ilvC-Gens von Escherichia coli ist bekannt (Wek and Hatfield, Journal of Biological Chemistry 261, 2441-2450 (1986)). Methoden zur Isolierung chromosomaler DNS sind ebenfalls bekannt (Sambrook et al.: Molecular Cloning, A Laboratory Manual (Cold Spring Harbor Laboratory Press, 1989). Durch Wahl geeigneter Primer kann das ilvC-Gen mit Hilfe der Polymerase-Kettenreaktion amplifiziert werden (Innis et al., PCR protocols. A guide to methods and applications, 1990, Academic Press). Anschließend wird es in einen Plasmidvektor eingefügt. Als Plasmidvektoren kommen solche in Frage, die in den entsprechenden Mikroorganismen replizieren können. Für Escherichia coli kommen z.B. die Vektoren pSC101 (Vocke and Bastia, Proceedings of the National Academy of Science U.S.A. 80 (21), 6557-6561 (1983)) oder pKK223-3 (Brosius and Holy, Proceedings of the National Academy of Science USA 81, 6929 (1984)), für Corynebacterium glutamicum z.B. der Vektor pJC1 (Cremer et al., Mol. Gen. Genet. 220:478-480 (1990)) oder pEKEx2 (Eikmanns et al., Gene 102:93-98 (1991)) oder pZ8-1 (Europäische Patentschrift 0 375 889) und für Saccharomyces cerevisiae z. B. der Vektor pBB116 (Berse, Gene 25: 109-117 (1983)) oder pDGl (Buxton et al., Gene 37: 207-214 (1985)) für die vorliegende Erfindung in Frage. Methoden zum Einbau von DNA-Fragmenten in Plasmidvektoren sind bei Sambrook et al.: Molecular Cloning, A Laboratory Manual (Cold Spring Harbor Laboratory Press, 1989) beschrieben. Methoden zur Transformation und Elektroporation sind bei Tauch et al. (FEMS Microbiology Letters 123:343-347 (1994)) beschrieben. Ein Beispiel eines solchen transformierten Stammes ist der Escherichia coli Stamm MG1655/pFE32. Plasmid pFE32 enthält das ilvC-Gen von MG1655 das in den Vektor pBR322 eingebaut wurde. Ein weiteres Beispiel eines solchen transformierten Stammes ist der Corynebacterium glutamicum Stamm ATCC13032/pFE91. Plasmid pFE91 enthält das ilvC-Gen von ATCC13032 das in den Vektor pECm3 eingebaut wurde. Das Plasmid pECm3 ist ein Derivat des Plasmids pECm2 (Tauch, 1994, FEMS Microbiological Letters, 123:343-348), dessen Kanamycin-Resistenzgen durch eine BglII- und BamHI-Restriktion mit anschließender Religation entfernt wurde.

Zum Einbau einer Mutation in das ilvC-Gen, die dessen Funktion ausschaltet, kann beispielsweise eine Deletion oder Insertion in dieses eingebaut werden. Zur Erzeugung einer Deletion kann mit Hilfe geeigneter Restriktionsenzyme und anschließender Verknüpfung der entstandenen Enden ein innerer Teil der Nukleotidsequenz des Strukturgens entfernt werden. Das auf diese Weise mutierte ilvC-Gen ist funktionslos. In gleicher Weise kann in das ilvC-Gen ein zweites Gen eingebaut werden, daß für eine Resistenz gegen ein Antibiotikum kodiert. Das auf diese Weise mutierte ilvC-Gen ist ebenfalls funktionslos. Das dergestalt mutierte ilvC-Gen kann anschließend in einen Mikroorganismus eingebracht und in dessen Chromosom gegen das Wildtyp-Gen ausgetauscht werden. Methoden, wie dieser Genaustausch durchzuführen ist, sind in der Literatur bekannt. Für Escherichia coli kann die von Hamilton et al. (Journal of Bacteriology 171, 4617-4622 (1989)) beschriebene Methode eingesetzt werden, die auf temperatursensitiven Replikationsmutanten des Plasmids pSC101 beruht. Ein derartiges Plasmid ist z. B. pMAK705. Für Corynebacterium glutamicum kann die von Schwarzer und Pühler (BIO/TECHNOLOGY 9, 84-87 (1991)) beschriebene Methode des Genaustauschs verwendet werden, bei der nichtreplikative Plasmidvektoren verwendet werden. Für Saccharomyces cerevisiae ist eine Methode des gezielten Genaustausches bei Roca et al. (Nucleic Acid Research 20(17), 4671-4672 (1992)) beschrieben.

Ein mutiertes ilvC-Gen kann beispielsweise folgendermaßen aus einem Wildtyp ilvC-Gen hergestellt werden. Das Plasmid pFE32 besteht aus pBR322 in dessen BamHI-Restriktionsschnittstelle das ilvC-Wildtypgen eingebaut wurde. In die KpnI-Schnittstelle des ilvC-Gens von pFE32 wurde das aacC1-Gen eingebaut, das für Resistenz gegen das Antibiotikum Gentamycin kodiert (Schweizer, BioTechniques 15 (5), 831-834 (1993)). Das auf diese Weise erhaltene Plasmid pFE33 enthält das ilvC::aacC1-Allel, das kein funktionsfähiges ilvC-Genprodukt mehr bilden kann. Das ilvC::aacC1-Allel wurde aus dem Plasmid pFE33 entnommen und in die SphI-Schnittstelle des Plasmids pMAK705 eingefügt, wodurch das Plasmid pDB1 entstand. Plasmid pDB1 ist ein zum Allelaustausch befähigter Plasmidvektor der zum einen aus pMAK705 und zum anderen aus dem ilvC::aacC1-Allel besteht. Plasmid pDB1 wurde nach der von Hamilton et al. beschriebenen Methode verwendet, um das in MG1655 vorhandene Wildtyp ilvC-Gen gegen das ilvC::aacC1-Allel auszutauschen. Der auf diese Weise entstandene Stamm wurde als FE4 bezeichnet.

Für die Isolierung einer Mutante von FE4, die eine Mutation im panE-Gen trägt wurde der Stamm FE4 einer Transposon-Mutagenese mit dem Transposon Tn5 unterzogen. Das Transposon Tn5 ist bei Auerswald (COLD SPRING HARBOR SYMPOSIA ON QUANTITATIVE BIOLOGY 45, 107-113 (1981)) beschrieben. Die Methode der Transposon-Mutagenese ist z. B. im Handbuch von Miller, A: Short Course in Bacterial Genetics, A Laboratory Manual and Handbook for Escherichia coli and Related Bacteria (Cold Spring Harbor Laboratory Press, 1992) beschrieben. Die Methode ist weiterhin bei Simon (Gene 80, 161-169 (1998)) und auch im Handbuch von Hagemann: Gentechnologische Arbeitsmethoden (Gustav Fischer Verlag, 1990) und zahlreichen anderen der Öffentlichkeit zugänglichen Publikationen beschrieben. Weiterhin können Mutanten auch nach Mutagenese mit ultraviolettem Licht oder nach Behandlung mit einer Mutations-auslösenden Chemikalie wie z. B. N-methyl-N'-nitro-N-nitrosoguanidin erzeugt werden. Unter den auf diese Weise erhaltenen Mutanten können nach Prüfung der Wuchsstoffbedürfnisse insbesondere des Pantothensäure-Bedürfnisses solche Mutanten isoliert werden, die eine Mutation in einem Gen der Pantothensäure-Biosynthese tragen. Von besonderem Interesse sind solche Pantothensäure bedürftigen Mutanten, die nicht Ketopantoat aber Pantoat als Wuchsstoff verwerten können und somit in dem für die Ketopantoat-Reduktase (EC 1.1.1169) kodierendem panE-Gen mutiert sind. Ein Beispiel hierfür ist der auf diese Weise erhaltene Stamm FE5, der neben der ilvC::aacC1-Mutation eine panE::Tn5-Mutation trägt.

Mikroorganismen, die eine Defektmutation im ilvC- und panE-Gen tragen wie beispielsweise der Escherichia coli Stamm FE5, können als Klonierwirte zur Isolierung des ilvC- und des besonders interessierenden panE-Gens oder von Nukleotidsequenzen, die für Proteine mit Ketopantoatreduktase-Aktivität kodieren, verwendet werden.

Hierzu wird von dem interessierenden Mikroorganismus eine Genbank angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine bekannte Genbank ist die des E. coli K-12 Stammes W3110, die von Kohara et al. (Cell 50, 495 - 508 (1987)) angelegt wurde. Genbanken verschiedener Mikroorganismen können mittlerweile käuflich erworben werden wie beispielsweise eine Genbank von Saccharomyces pombe Stamm Sp63 der Firma Stratagene (Heidelberg, Deutschland) im Plasmid Lambda FIX II (Elgin, Strategies 4: 6-7(1991)), eine Genbank des Escherichia coli Stammes W1485 der Firma CLONTECH (Heidelberg, Deutschland) im Plasmid pGAD10 (Kitts, CLONTECH (Heidelberg, Deutschland) Vectors On Disc version 1.3, 1994), dessen Nukleotdsequenz unter der GenBank accession number U13188 zugänglich ist. Die auf die oben beschriebene Art und Weise hergestellte Genbank kann dann in den oben beschriebenen Wirt FE5 durch Transformation eingebracht werden. So wurde beispielhaft die pGAD10 - Genbank von W1485 in den Stamm FE5 durch Transformation eingebracht und die erhaltenen Transformanten auf ihre Fähigkeit untersucht auf einem Pantothensäure freiem Nährboden zu wachsen. Die in der Plasmid-DNA der erhaltenen Pantothensäure prototrophen Transformanten enthaltenen Insertionen kann durch Bestimmung der Nukleotidsequenz untersucht werden. Methoden zur Bestimmung von Nukleotidsequenzen können beispielsweise bei Sanger et al. (Proceedings of the National Academy of Science USA 74:5463-5467 (1977)) nachgelesen werden. Nukleotidsequenzen können Genen mittels Homologieuntersuchungen zugeordnet werden. Eine Möglichkeit für diese Homologiesuche bietet der Vergleich mit Nukleotidsequenzen der EMBL und GenBank Datenbanken, die mittels des BLAST E-mail Service (Altschul, Journal of Molecular Biology 215, 403-410 (1990)) durchgeführt werden können. Ein Beispiel einer solchen Transformante ist der Escherichia coli Stamm FE5/pFEbank16 der das panE-Gen des E. coli Stammes MG1655 trägt.

Das auf die beschriebene Weise isolierte und identifizierte panE-Gen kann anschließend in einem gewünschten Mikroorganismus zur Expression gebracht werden. Hierzu wird es durch Plasmidvektoren amplifiziert. Diese wiederum können mit Signalstrukturen ausgerüstet sein, die für eine effiziente Transkription und Translation sorgen. Eine Übersicht über Expressionsvektoren findet man beispielsweise in dem Lehrbuch von Winnacker:Gene und Klone, Eine Einführung in die Gentechnologie (Verlag Chemie, Weinheim, Deutschland, 1990) oder bei Sambrook et al.: Molecular Cloning, A Laboratory Manual (Cold Spring Harbor Laboratory Press, 1989). Weiterhin können Expressionssignale wie z. B. der tac-Promotor stromaufwärts des panE-Gens in das Chromosom eingebaut werden. Derartige Methoden sind in WO 98/04715 beschrieben. Das zu exprimierende panE-Gen kann aus dem klonierten chromosomalen DNA - Fragment entnommen werden oder es kann wiederum mit Hilfe der Polymerase - Kettenreaktion amplifiziert werden. Die in dem betreffenden Mikroorganismus vorhandene Menge an Ketopantoat-Redukase kann mit Hilfe des von Shimizu et al. (Journal of Biological Chemistry 263: 12077-12084 (1988)) beschriebenen Methode bestimmt werden. Ein Beispiel eines derartigen Stammes ist der Escherichia coli Stamm MG1655/pFE65. Plasmid pFE65 besteht aus dem Vektor pKK223-3 in dessen EcoRI-Restriktionsschnittstelle das panE-Gen von Escherichia coli MG1655 eingebaut wurde.

Erfindungsgemäß hat es sich als vorteilhaft erwiesen, zusätzlich zu dem für die Ketopantoat-Reduktase kodierenden panE-Gen eines oder mehrere Gene der Pantothensäure-Biosynthese zu überexprimieren. Hierzu gehören die Gene die für die Enzyme Ketopantoat-Hydroxymethyltransferase (EC 4.1.2.12), Aspartat-1-Decarboxylase (EC 4.1.1.11) und Pantothenat-Synthetase (EC 6.3.2.1) kodieren. In Escherichia coli tragen diese Gene die Bezeichnung panB, panD und panC (Miller, A Short Course in Bacterial Genetics, A Laboratory Manual and Handbook for Escherichia coli and Related Bacteria (Cold Spring Harbor Laboratory Press, 1992)). Hierzu können die Gene in verschiedene kompatible Plasmidvektoren eingebaut werden. Beispiele hierfür sind bei Bartolome et al. (Gene 102, 75-78 (1991) beschrieben. Weiterhin kann die Genexpression durch Veränderung der stromaufwärts gelegenen chromosomalen Signalstrukturen erhöht werden. Weiterhin können die betreffenden Gene nacheinander angeordnet unter die Kontrolle eines gemeinsamen Promotors gestellt und in einen Plasmidvektor eingebaut und in einen geeigneten Mikroorganismus eingeführt werden. Ein Beispiel hierfür ist der Escherichia coli Stamm MG1655/pFE80. Das Plasmid pFE80 besteht aus dem Plasmid pKK223-3, das die Gene panB, panD, panC und panE in der angegebenen Reihenfolge enthält. Stromaufwärts des panB-Gens ist in pFE80 der tac-Promotor als Expressionssignal enthalten.

Es hat sich weiterhin als vorteilhaft erwiesen, das panE-Gen und die Expressionseinheit bestehend aus den Genen panB, panD, panC und panE in Wirtsstämmen zu überexprimieren, die chromosomale Mutationen enthalten.

Es können Mutationen einzeln oder gemeinsam verwendet werden, die Resistenzen gegen Stoffwechselprodukte wie z. B. L-Valin oder α-Ketoisovaleriansäure oder gegen Analoga von Stoffwechselprodukten wie z. B. β-Hydroxyasparaginsäure oder O-Methylthreonin bewirken. Derartige Mutanten treten spontan auf oder können nach Mutagenese mit ultraviolettem Licht oder nach Behandlung mit einer Mutations-auslösenden Chemikalie wie z. B. N-methyl-N'-nitro-N-nitrosoguanidin erzeugt werden und anschließend auf Agarplatten, die die entsprechenden Substanz enthalten, selektioniert werden. Verfahren zur Mutationsauslösung und Selektion sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA) nachgelesen werden. Ein Beispiel für eine derartige Mutante ist der Escherichia coli Stamm FE6, die als spontan auftretende, gegen L-Valin resistente Mutante des Stammes MG1655 isoliert wurde.

Weiterhin können gezielt ungünstige oder störende, chromosomal kodierte Stoffwechselreaktionen ausgeschaltet werden. Hierzu werden in die entsprechenden Gene Insertionen oder Deletionen eingefügt und die auf diese Weise entstandenen mutierten Gene beziehungsweise Allele in das Chromosom des betreffenden Wirtes eingebaut. Es können die Methoden eingesetzt werden, die oben für die Mutation des ilvC-Gens beschrieben worden sind. Ein Beispiel für eine derartige Mutante ist der Escherichia coli Stamm FE7, der eine avtA::aadB-Mutation im Chromosom trägt. Hierbei handelt es sich um den Stamm MG1655 in dessen avtA-Gen das Resistenz gegen Streptomycin vermittelnde aadB-Gen aus Plasmid pHP45Ω (Prentki und Krisch, Gene 29, 303-313 (1984)) eingesetzt wurde.
In den auf diese Weise hergestellten Wirtsstämmen kann dann das panE-Gen allein oder in Kombination mit anderen Genen überexprimiert werden. Beispiele hierfür sind die Stämme FE6/pFE80 und FE7/pFE80.

Die erfindungsgemäß hergestellten Mikroorganismen können kontinuierlich oder diskontinuierlich im batch - Verfahren oder im fed batch (Zulaufverfahren) oder repeated fed batch Verfahren (repetitives Zulaufverfahren) zum Zwecke der Pantothensäure-Produktion kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.
Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlenhydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies Vorstufen der Pantothensäure wie β-Alanin oder Ketopantoinsäure und deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak, oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester oder Silikonöle eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 50°C und vorzugsweise bei 25°C bis 45°C. Die Kultur wird solange fortgesetzt bis sich ein Maximum an Pantothensäure gebildet hat. Dieses Ziel wird normalerweise innerhalb von 10 Stunden bis 160 Stunden erreicht.

Die Konzentration an gebildeter Pantothensäure kann mit bekannten Verfahren (Velisek; Chromatographic Science 60, 515-560 (1992)) bestimmt werden.

Folgende Mikroorganismen wurden bei der Deutschen Sammlung für Mikrorganismen und Zellkulturen (DSMZ, Braunschweig, Deutschland) gemäß Budapester Vertrag hinterlegt:
- Escherichia coli K12 Stamm FE5 als DSM12378
- Escherichia coli K12 Stamm MG1655/pFE32 als DSM12413
- Escherichia coli K12 Stamm MG1655/pFE65 als DSM12382
- Escherichia coli K12 Stamm MG1655/pFE80 als DSM12414
- Escherichia coli K12 Stamm FE6 als DSM12379
- Escherichia coli K12 Stamm FE7 als DSM12380

Mit dem erfindungsgemäßen Verfahren wird dem Fachmann ein neues Werkzeug an die Hand gegeben, um die Pantothensäurebildung von Mikroorganismen gezielt zu verbessern.

### Beispiele

Die vorliegende Erfindung wird im folgenden anhand von Ausführungsbeispielen näher erläutert.

### Beispiel 1

### Herstellung einer ilvC::aacC1 panE::Tn5 Mutante von Escherichia coli K12 Stamm MG1655

### 1. Herstellung der ilvC::aacC1 Mutante

Ausgehend von der Nukleotidsequenz für das ilvC-Gen in E. coli K12 MG1655, (EMBL-GenBank: Accession Nr. M87049) wurden PCR-Primer synthetisiert (MWG Biotech (Ebersberg, Deutschland)). Ein ca. 1500 bp großes DNA-Fragment konnte mit diesen Primern unter der Standard-PCR-Methode von Innis et al. (PCR protocols. A guide to methods and applications, 1990, Academic Press) amplifiziert werden. Die für die PCR eingesetzte chromosomale E. coli K12 MG1655 DNA wurde mittels des NucleoSpin C + T Kits (Macherey-Nagel (Düren, Deutschland), Produktbeschreibung NucleoSpin C + T, Art.-Nr. 740952) isoliert. Die Größe wurde durch gelelektrophoretische Auftrennung (30 Minuten, 10V/cm) in einem 0,8 %igen Agarosegel bestimmt.

PCR-Primer für das ilvC-Gen aus E. coli:

| | | |
|---|---|---|
| ilvC1 | 5' - AGAAGCACAACATCACGAGG | -3' |
| ilvC2 | 5' - CTCCAGGAGAAGGCTTGAGT | -3' |

Das PCR-Produkt des ilvC-Gens wurde in das Plasmid pCR®2.1 und in den E. coli Stamm TOP10F' transformiert (Invitrogen (Leek, Niederlande), Produktbeschreibung Original TA Cloning® Kit, Cat. no. KNM2030-01).

Der Klonierungserfolg wurde durch Spaltung der DNA des Plasmids pCR®2.1ilvC mit den Restriktionsenzymen EagI (Pharmacia Biotech (Freiburg, Deutschland), Produktbeschreibung EagI, Code no. 27-0885-01), EcoRI (Pharmacia Biotech (Freiburg, Deutschland), Produktbeschreibung EcoRI, Code no. 27-0884-03) und KpnI (Pharmacia Biotech (Freiburg, Deutschland), Produktbeschreibung KpnI, Code no. 27-0908-01) nachgewiesen. Dazu wurde die Plasmid DNA mittels des QIAprep Spin Plasmid Kits (QIAGEN (Hilden, Deutschland), Cat. no. 27106) isoliert und nach Spaltung in einem 0,8 %igen Agarosegel aufgetrennt (30 Minuten, 10V/cm).

Zur Isolation des ilvC-Gens aus dem Plasmid pCR®2.1ilvC wurde die isolierte Plasmid DNA mit den Enzymen HindIII (Pharmacia Biotech (Freiburg, Deutschland), Produktbeschreibung HindIII, Code no. 27-0860-01) und XbaI (Pharmacia Biotech (Freiburg, Deutschland), Produktbeschreibung XbaI, Code no. 27-0948-01) gespalten, der Spaltungsansatz im 0,8 %igen Agarosegel aufgetrennt (30 Minuten, 10V/cm) und das 1,5 kbp ilvC-Fragment mit Hilfe des GLASSMAX™ Kits isoliert (GIBCO BRL (Eggenstein, Deutschland), Produktbeschreibung GLASSMAX™ Spin Cartridges, Cat. no. 15590-052). Das isolierte ilvC-Fragment wurde mit dem ebenfalls HindIII und XbaI gespaltenen Plasmid pMAK705 (Hamilton et al., Journal of Bacteriology 1989, 171: 4617-4622) mittels T4 DNA Ligase (Pharmacia Biotech (Freiburg, Deutschland), Produktbeschreibung T4 DNA Ligase, Code no. 27-0870-03) ligiert und der E. coli Stamm DH5αmcr (Grant, Proceedings of the National Academy of Science 1990, 87: 4645-4649) mit dem Ligationsansatz elektroporiert (Tauch, FEMS Microbiology Letters 1994, 123: 343-347). Die Selektion auf Plasmid tragende Zellen erfolgte durch ausplattieren des Elektroporationsansatzes auf LB Agar (Lennox, Virology' 1955, 1: 190), welcher mit 25 µg/ml Chloramphenicol (Sigma (Deisenhofen, Deutschland) (Deisenhofen, Deutschland), Code no. C 0378) versetzt war und 24 Stunden Inkubation bei 30°C. Das gesuchte Plasmid konnte nach DNA-Isolation und Kontrollspaltung, bei anschließender Gelelektrophorese im 0,8 %igen Agarosegel (30 Minuten, 10V/cm), mit den Enzymen HindIII, XbaI und KpnI in einem Klon identifiziert werden und wurde mit pFE30 bezeichnet.

Zur Isolation des ilvC-Gens aus dem Plasmid pFE30 wurde die isolierte Plasmid DNA mit dem Enzym BamHI (Pharmacia Biotech (Freiburg, Deutschland), Produktbeschreibung BamHI, Code no. 27-0868-03) gespalten, der Spaltungsansatz im 0,8 %igen Agarosegel aufgetrennt (30 Minuten, 10V/cm) und das 1,5 kbp ilvC-Fragment mit Hilfe des GLASSMAX™ Kits isoliert. Das isolierte ilvC-Fragment wurde mit dem ebenfalls BamHI gespaltenen Plasmid pBR322 (Sutcliffe, COLD SPRING HARBOR SYMPOSIA ON QUANTITATIVE BIOLOGY 1979, 43: 77-90) mittels T4 DNA Ligase ligiert und der E. coli Stamm DH5αmcr mit dem Ligationsansatz elektroporiert. Die Selektion auf Plasmid tragende Zellen erfolgte durch ausplattieren des Elektroporationsansatzes auf LB Agar, welcher mit 100 µg/ml Ampicillin (Sigma (Deisenhofen, Deutschland), Code no. A 9518) versetzt war und 24 Stunden Inkubation bei 37°C. Erhaltene Kolonien wurden parallel auf LB+Ampicillin-Agar und LB+(5µg/ml)Tetracyclin (Sigma (Deisenhofen, Deutschland), Code no. T3383) angeimpft. DNA aus Tetracyclin sensitiven Kolonien wurde mit dem QIAprep Spin Plasmid Kit isoliert und der Erfolg der Klonierung mittels einer BamHI- und KpnI-Spaltung und anschließender Auftrennung im 0,8 %igen Agarosegel (30 Minuten, 10V/cm) verifiziert. Das konstruierte Plasmid wurde pFE32 genannt.

In die KpnI-Schnittstelle des Plasmids pFE32 wurde ein aacC1-Gen kloniert und das erhaltene Plasmid mit pFE33 bezeichnet. Das aacC1-Gen wurde dazu aus einem Agarosegel (30 Minuten, 10V/cm) isoliert, in welchen ein KpnI-Restriktionsansatz des Plasmids pMS255 (Becker, Gene 1995, 162: 37-39) aufgetrennt wurde. Ligation erfolgte mit T4-DNA-Ligase. Nach Elektroporation des Ligationsansatzes in den Stamm DH5αmcr wurden die Transformanten auf PA-Agar (Sambrook, Molecular cloning, 2^{nd} edn, Cold Spring Harbor, 1989), welcher mit 10 µg/ml Gentamycin (Sigma (Deisenhofen, Deutschland), Code no. G3632) versetzt war, selektioniert. DNA aus Gentamycin resistenten Kolonien wurde mit dem QIAprep Spin Plasmid Kit isoliert und der Erfolg der Klonierung mittels einer BamHI- und KpnI-Spaltung und anschließender Auftrennung im 0,8 %igen Agarosegel (30 Minuten, 10V/cm) verifiziert.

Das ilvC::aacC1-Fragment wurde aus dem Plasmid pFE33 mittels SphI-(Pharmacia Biotech (Freiburg, Deutschland), Produktbeschreibung SphI, Code no. 27-0951-01) Restriktion gespalten, im 0,8 %igen Agarosegel (30 Minuten, 10V/cm) aufgetrennt und mit dem GLASSMAX™ Kit isoliert. Das Fragment wurde mit dem SphI gespaltenem Plasmid pMAK705 mittels T4-DNA-Ligase ligiert, der Ligationsansatz in den Stamm DH5αmcr elektroporiert und Transformanten durch Inkubation auf PA+Gentamycin-Agar für 24 Stunden bei 30°C selektioniert. DNA aus Gentamycin resistenten Kolonien wurde mit dem QIAprep Spin Plasmid Kit isoliert und der Erfolg der Klonierung mittels einer SphI- und EcoRI-Spaltung im 0,8 %igen Agarosegel (30 Minuten, 10V/cm) nachgewiesen. Das konstruierte Plasmid wurde mit pDB1 bezeichnet.

Mit Hilfe des Plasmids pDB1 wurde in dem Stamm E. coli K12 MG1655 das chromosomale ilvC-Gen gegen das unterbrochene ilvC::aacC1-Fragment ausgetauscht. Für den Genaustausch wurde eine modifizierte Methode nach Hamilton et al. verwendet. Das Plasmid pDB1 wurde in den E. coli K12 MG1655 Stamm elektroporiert, anschließend wurden die Transformanten zur Selektion auf Cointegrate auf LB-Chloramphenicol-Agar bei 42°C für 24 Stunden inkubiert. Die erhaltenen Kolonien wurden zur Vereinzelung wiederum auf dem gleichen Medium ausgestrichenund bei 42°C für 24 Stunden inkubiert. Zur Desintegration des Plasmids wurden Einzelkolonien in 5ml LB-Flüssigmedium bei 42°C für 24 Stunden inkubiert, und anschließend eine Verdünnungsreihe des Flüssigmediums auf LB-Chloramphenicol-Agar ausplattiert. Diese Verdünnungsreihe wurde bei 30°C für 24 Stunden inkubiert. Zur Kurierung vom Plasmid wurden aus der Verdünnungsreihe erhaltene Einzelkolonien in 3 aufeinanderfolgenden Einzelkolonieausstrichen auf LB-Agar bei 42°C für jeweils 24 Stunden angezogen. Zur Kontrolle des Phänotyps wurden die erhaltenen Einzelkolonien parallel auf Agarplatten mit folgenden Medien angeimpft: Medium E (Vogel, Journal of Biological Chemistry 1956, 218: 97-106) + Glucose (0,4%), MediumE + Glucose (0,4%) (Sigma (Deisenhofen, Deutschland), Code no. G8270)+ 50µg/ml Isoleucin (Sigma (Deisenhofen, Deutschland), Code no. 17268), MediumE + Glucose (0,4%) + 50µg Ketoisovalerat (ICN (Eschwege, Deutschland), Code no. 151395), MediumE + Glucose (0,4%) + 50µg/ml Isoleucin+50µg Ketoisovalerat, PA-Medium + Gentamycin und LB-Medium + Chloramphenicol. Diese Medien wurden für 48 Stunden bei 37°C inkubiert. Unter 150 getesteten Einzelkolonien befand sich eine deren Phänotyp den Austausch des chromosomalen ilvC-Gens durch das ilvC::aacC1-Fragment anzeigte. Dieser Stamm wurde mit FE4 bezeichnet.

### 2. Herstellung der ilvC::aacC1 panE::Tn5 Doppelmutante

Der Stamm FE4 wurde in 5ml LB-Flüssigmedium + 10mM MgSO₄ + 0,2% Maltose (Sigma (Deisenhofen, Deutschland), Code no. M5885) (LBMgMal) bei 37°C bis zu einer optischen Dichte von 0,5 angezogen. Die Messung der optischen Dichte efolgte mit einem Pharmacia (Freiburg, Deutschland) Novaspec II Photometer bei einer Wellenlänge von 660 nm. 2ml der Bakterienlösung wurden 5min bei 3000rpm abzentrifugiert (Beckmann Model J2-21 Centrifuge, Rotor JA-17). Nach Aufnahme des Pellets mit 0,5ml LBMgMal- Flüssigmedium wurde die Suspension mit 30µl λ::Tn5(Simon, Gene 1989, 80(1):161-169)-Lysat, ca. 10⁸ Bakteriophagen, versetzt. Dieses Lysat wurde aus dem Stamm E. coli K12 C600 (Appleyard, Genetics 1954, 39:440-452) isoliert, nach der Methode von Hagemann (Gentechnologische.Arbeitsmethoden, Gustav Fischer Verlag,1990: 14-18).Die Suspension mit dem λ::Tn5-Lysat wurde für 45 Minuten bei 30°C inkubiert. Nach Abzentrifugation bei 3000rpm für 5 Minuten wurde das Pellet in 10ml PA + 10mM Pyrophosphat aufgenommen und für 3 Stunden bei 37°C inkubiert. Die Bakterienlösung wurde als Verdünnungsreihe auf Medium E-Agar + Glucose (0,4%) + 25µg/ml Kanamycin + 50µg/ml Isoleucin + 50µg/ml Ketoisovalerat + 50µg/ml Pantothenat ausplattiert und bei 37°C für 48 Stunden inkubiert. Einzelkolonien wurden parallel auf MediumE-Agar + Glucose (0,4%) + 25µg/ml Kanamycin + 50µg/ml Isoleucin + 50µg/ml Ketoisovalerat + 50µg/ml Pantothenat und auf Medium E-Agar + Glucose (0,4%) + 25µg/ml Kanamycin + 50µg/ml Isoleucin + 50µg/ml Ketoisovalerat angeimpft und bei37°C für 48 Stunden inkubiert. Unter 14000 angeimpften Einzelkolonien konnte eine, als FE5 bezeichnete Kolonie, identifiziert werden welche auf Medium E-Agar + Glucose (0,4%) + 25µg/ml Kanamycin + 50µg/ml Isoleucin + 50µg/ml Ketoisovalerat + 50µg/ml Pantothenat wuchs, nicht aber auf Medium E-Agar + Glucose (0,4%) + 25µg/ml Kanamycin + 50µg/ml Isoleucin + 50µg/ml Ketoisovalerat.

### 3. Charakterisierung der Stämme FE4 und FE5

Zusammen mit den E. coli Stämmen SJ2 (Jakowski, Genetic Stock Center, Yale University),der eine Mutation im panB-Gen trägt, MW6 (Williams, Genetic Stock Center, Yale University), der eine Mutation im panC-Gen trägt, und DV9 (Vallari, Journal of Bacteriology 1985, 164:136-142), der eine Mutation im panD-Gen trägt, sowie einem Wildtyp wurden die Stämme FE4 und FE5 auf unterschiedlich supplementierten Grundmedien (Medien E-Agar + Glucose (0,4%) + 50µg/ml Isoleucin + 50µg/ml Ketoisovalerat; bei SJ2, DV9 und MW6 zusätzlich 50 µg/ml Thiamin) ausgestrichen und bei 37°C für 48 Stunden inkubiert. Als zusätzliche Supplemente wurden Pantothenat (Calcium-Salz), Ketopantoat (Natrium- Salz), β-Alanin (Sigma (Deisenhofen, Deutschland), Code no. A7752) und Pantoat (Kalium-Salz) verwendet. Ketopantoat wurde aus Ketopantolacton durch Behandlung mit äquimolaren Mengen an NaOH bei 60°C und anschließendem Eindampfen hergestellt. Ketopantolacton wurde nach Ojima et al. (Organic Synthesis 63, 18 (1985)) synthetisiert. Pantoat wurde aus Pantoyllacton (Sigma (Deisenhofen, Deutschland), Code no. P2625) nach der Methode von Primerano und Burns (Journal of Bacteriology 1983, 153: 259-269) hergestellt. Das Ergebnis des Wachstumstests (Tabelle 1) zeigte, daß der Stamm FE4 auf allen unterschiedlich supplementierten Grundmedien wuchs. Der Stamm FE5 wuchs nur auf Medien, die entweder mit Pantothenat oder Pantoat supplementiert waren, nicht aber auf Grundmedien die mit Ketopantoat versetzt waren.

**Tabelle 1**

| Stamm | Supplemente des Grundmediums | | | | |
|---|---|---|---|---|---|
| | keine | β-Alanin [50µg/ml] | Ketopantoat [50µg/ml] | Pantoat [50µg/ml] | Pantothenat [50µg/ml] |
| | | | | | |
| MG1655 | + | + | + | + | + |
| | | | | | |
| SJ2 | - | - | + | + | + |
| | | | | | |
| MW6 | - | - | - | - | + |
| | | | | | |
| DV9 | - | + | - | - | + |
| | | | | | |
| FE4 | + | + | + | + | + |
| | | | | | |
| FE5 | - | - | - | + | + |

| | | | | | |
|---|---|---|---|---|---|
| + = Wachstum - = kein Wachstum | | | | | |

### Beispiel 2

### Isolierung des panE-Gens von Escherichia coli K12 Stamm W1485 (Teil der Beschreibung)

In den Stamm FE5 wurde die E. coli K12 W1485 MATCHMAKER Genomic Library (CLONTECH (Heidelberg, Deutschland), Cat. no. XL4001AB) elektroporiert. Die E. coli K12 MATCHMAKER Genomic Library enthält die chromosomale DNA von E. coli K12 W1485 als durchschnittlich 1,0 kbp große Inserts in dem Plasmid pGAD10, die Größe der einzelnen Inserts variiert dabei von 0,5 - 3,0 kbp (CLONTECH (Heidelberg, Deutschland)). Die Selektion der Transformanten erfolgte durch Ausplattieren auf Medium E-Agar + Glucose (0,4%) + 100 µg/ml Ampicillin + 50µg/ml Isoleucin + 50µg/ml Ketoisovalerat. Aus 20 erhaltenen Kolonien wurde mit Hilfe des QIAprep Spin Plasmid Kits die Plasmid DNA isoliert. Durch eine EcoRI-Spaltung der Plasmid DNA und anschließender Auftrennung im 0,8 %igen Agarosegel (30 Minuten, 10V/cm) wurde gezeigt, daß es sich bei den Plasmiden um 20 pGAD10 Vektoren mit unterschiedlich großen Inserts handelte. Die Sequenzierung (IIT Biotech (Bielefeld, Deutschland)) der Inserts ergab, durch Homologievergleiche mit dem BLAST-Programm (Altschul, Journal of Molecular Biology 1990, 215: 403-410), daß die Inserts in 7 Fälle ein vollständiges ilvC-Gen und in 13 Fällen ein offenes Leseraster enthielten, der mit "ähnlich zu Salmonella typhimurium apbA" bezeichnet wurde (EMBL-GenBank: Accession Nr. U82664). Dieser offenes Leseraster wurde mit panE bezeichnet.

### Beispiel 3

### Überexpression des ilvC-Gens von E. coli in E. coli K12 Stamm MG1655

Zur Überexpression des ilvC Gens wurde das Plasmid pFE32 (siehe Beispiel 1)verwendet. Die Kodierregion des ilvC-Gens steht im Plasmid pFE32 unter der Kontrolle des vom Plasmid pBR322 kodierten tet-Promotors. Das Plasmid pFE32 wurde in den Stamm E. coli K12 MG1655 elektroporiert und Transformanten auf LB Agar, welcher mit 100µg/ml Ampicillin versetzt war, nach anschließender Inkubation für 24 Stunden bei 37°C selektioniert. Der erhaltene Stamm wurde als MG1655/pFE32 bezeichnet.

### Beispiel 4

### Überexpression des panE-Gens von E. coli in E. coli K12 Stamm MG1655

Ausgehend von der Nukleotidsequenz für das panE-Gen in E. coli K12 MG1655 wurden PCR-Primer synthetisiert (MWG Biotech (Ebersberg, Deutschland)). Ein ca. 1000 bp großes DNA-Fragment konnte mit diesen Primern unter der Standard-PCR-Methode aus chromosomaler E. coli K12 MG1655 DNA amplifiziert werden. Die für die PCR eingesetzte chromosomale E. coli K12 MG1655 DNA wurde mittels des NucleoSpin C + T Kits isoliert Die Größe wurde durch gelelektrophoretische Auftrennung (30 Minuten, 10V/cm) in einem 0,8 %igen Agarosegel bestimmt.

PCR-Primer für das panE-Gen aus E. coli:

| | | |
|---|---|---|
| panE1 | 5' - AGGAGGACAATGAAAATTAC | -3' |
| panE2 | 5' - TCAGTCTCTTCACTACCAGG | -3' |

Das PCR-Produkt des panE-Gens wurde in das Plasmid pCR®2.1 und in den E. coli Stamm TOP10F' transformiert (Invitrogen (Leek, Niederlande), Produktbeschreibung Original TA Cloning® Kit, Cat. no. KNM2030-01). Der Klonierungserfolg wurde durch Spaltung der DNA des Plasmids pCR®2.1panE mit den Restriktionsenzymen EcoRI und HincII (Pharmacia Biotech (Freiburg, Deutschland), Produktbeschreibung HincII, Code no. 27-0858-01) nachgewiesen. Dazu wurde die Plasmid DNA mittels des QIAprep Spin Plasmid Kits isoliert und nach Spaltung in einem 0,8 %igen Agarosegel aufgetrennt (30 Minuten, 10V/cm).

Zur Isolation des panE-Gens aus dem Plasmid pCR®2.1panE wurde die isolierte Plasmid DNA mit dem Enzym EcoRI gespalten, der Spaltungsansatz im 0,8 %igen Agarosegel aufgetrennt (30 Minuten, 10V/cm) und das 1,0 kbp panE-Fragment mit Hilfe des GLASSMAX™ Kits isoliert. Das isolierte panE-Fragment wurde mit dem ebenfalls EcoRI gespaltenen Plasmid pKK223-3 mittels T4 DNA Ligase ligiert und der E. coli Stamm DH5αmcr mit dem Ligationsansatz elektroporiert. Die Selektion auf Plasmid tragende Zellen erfolgte durch Ausplattieren des Elektroporationsansatzes auf LB Agar, welcher mit 100µg/ml Ampicillin versetzt war und anschließender Inkubation für 24 Stunden bei 37°C. Das gesuchte Plasmid konnte nach DNA-Isolation und Kontrollspaltung, bei anschließender Gelelektrophorese im 0,8 %igen Agarosegel (30 Minuten, 10V/cm), mit den Enzymen EcoRI und HincII in einem Klon identifiziert werden und wurde mit pFE65 bezeichnet.

Die Kodierregion des panE-Gens steht im Plasmid pFE65 unter der Kontrolle des vom Plasmid pKK223-3 kodierten tac-Promotors. Das Plasmid pFE65 wurde in den Stamm E. coli K12 MG1655 elektroporiert und Transformanten auf LB Agar, welcher mit 100µg/ml Ampicillin versetzt war, und anschließender Inkubation für 24 Stunden bei 37°C selektioniert. Der erhaltene Stamm wurde als E. coli K12 MG1655/pFE65 bezeichnet.

### Beispiel 5

### Überexpression des panE-Gens von E. coli zusammen mit panB, panC und panD von E. coli in E. coli K12 Stamm MG1655

Ausgehend von der Nukleotidsequenz für das panB-Gen, panC-Gen und panD-Gen in E. coli K12 MG1655 (EMBL-GenBank: Accession Nr. L17086) wurden PCR-Primer synthetisiert (MWG Biotech (Ebersberg, Deutschland)). Mit den panB Primern konnte ein ca. 800 bp großes DNA-Fragment, mit den panD Primern ein ca. 400 bp großes DNA-Fragment unter der Standard-PCR-Methode aus chromosomaler E. coli K12 MG1655 DNA amplifiziert werden. Mit den panC Primern konnte ein ca. 850 bp großes DNA-Fragment mittels einer modifizierten Standard-PCR-Methode aus chromosomaler E. coli K12 MG1655 DNA amplifiziert werden. Die Taq-Polymerase wurde durch die Pfu-Polymerase ersetzt und die Pufferbedingungen im PCR-Ansatz entsprechend modifiziert (STRATAGENE (Heidelberg, Deutschland), Produktbeschreibung Pfu-Polymerase, Code no. 600135). Die für die PCR eingesetzte chromosomale E. coli K12 MG1655 DNA wurde mittels des NucleoSpin C + T Kits isoliert Die Größe aller Amplifikate wurde durch gelelektrophoretische Auftrennung (30 Minuten, 10V/cm) in einem 0,8 %igen Agarosegel bestimmt.

PCR-Primer für das panB-Gen aus E. coli:

| | | |
|---|---|---|
| panB1 | 5' - AGGATACGTTATGAAACCGA | -3' |
| panB2 | 5' - ACAACGTGACTCCTTAATGG | -3' |

PCR-Primer für das panC-Gen aus E. coli:

| | | |
|---|---|---|
| panC1 | 5' - AGGAGTCACGTTGTGTTAAT | -3' |
| panC2 | 5' - AAGTATTACGCCAGCTCGAC | -3' |

PCR-Primer für das panD-Gen aus E. coli:

| | | |
|---|---|---|
| panD1 | 5' - AGGTAGAAGTTATGATTCGC | -3' |
| panD2 | 5' - TAACAATCAAGCAACCTGTA | -3' |

Das PCR-Produkt des panB-Gens wurde in das Plasmid pCR®2.1 und in den E. coli Stamm TOP10F' transformiert (Invitrogen (Leek, Niederlande)). Der Klonierungserfolg des panB-PCR-Produktes wurde durch Spaltung der DNA des Plasmids PCR®2.1panB mit den Restriktionsenzymen EcoRI, EcoRV (Pharmacia Biotech (Freiburg, Deutschland), Produktbeschreibung EcoRV, Code no. 27-0934-01) und PvuII (Pharmacia Biotech (Freiburg, Deutschland), Produktbeschreibung PvuII, Code no. 27-0960-01) nachgewiesen. Dazu wurde die Plasmid DNA mittels des QIAprep Spin Plasmid Kits isoliert und nach Spaltung in einem 0,8 %igen Agarosegel aufgetrennt (30 Minuten, 10V/cm). Das PCR-Produkt des panD-Gens wurde in das Plasmid pCR®2.1 und in den E. coli Stamm TOP10F' transformiert (Invitrogen (Leek, Niederlande)). Der Klonierungserfolg des panD-PCR-Produktes wurde durch Spaltung der DNA des Plasmids pCR®2.1panD mit den Restriktionsenzymen EcoRI, EcoRV und HincII nachgewiesen. Dazu wurde die Plasmid DNA mittels des QIAprep Spin Plasmid Kits isoliert und nach Spaltung in einem 0,8 %igen Agarosegel aufgetrennt (30 Minuten, 10V/cm). Das PCR-Produkt des panC-Gens wurde in das Plasmid pUC19 (Viera, Gene 1982 19:259-268) und in den E. coli Stamm DH5αmcr elektroporiert. Der Klonierungserfolg des panC-PCR-Produktes wurde durch Spaltung der DNA des Plasmids pUC19panC mit den Restriktionsenzymen EcoRI, HindIII und SalI (Pharmacia Biotech (Freiburg, Deutschland), Produktbeschreibung SalI, Code no. 27-0882-01) nachgewiesen. Dazu wurde die Plasmid DNA mittels des QIAprep Spin Plasmid Kits isoliert und nach Spaltung in einem 0,8 %igen Agarosegel aufgetrennt (30 Minuten, 10V/cm). Das konstruierte Plasmid wurde pFE60 genannt.

Zur Isolation des panB-Gens aus dem Plasmid pCR®2.1panB wurde die isolierte Plasmid DNA mit dem Enzym EcoRI gespalten, der Spaltungsansatz im 0,8 %igen Agarosegel aufgetrennt (30 Minuten, 10V/cm) und das 800 bp panB-Fragment mit Hilfe des GLASSMAX™ Kits isoliert. Das isolierte panB-Fragment wurde mit dem ebenfalls EcoRI gespaltenen Plasmid pKK223-3 mittels T4 DNA Ligase ligiert und der E. coli Stamm DH5αmcr mit dem Ligationsansatz elektroporiert. Die Selektion auf Plasmid tragende Zellen erfolgte durch Ausplattieren des Elektroporationsansatzes auf LB Agar, welcher mit 100µg/ml Ampicillin versetzt war und anschließender Inkubation für 24 Stunden bei 37°C. Das gesuchte Plasmid konnte nach DNA-Isolation und Kontrollspaltung, bei anschließender Gelelektrophorese im 0,8 %igen Agarosegel (30 Minuten, 10V/cm), mit den Restriktionsenzymen EcoRI, EcoRV und PvuII in einem Klon identifiziert werden und wurde mit pFE40 bezeichnet. Die Kodierregion des panB-Gens steht im Plasmid pFE40 unter der Kontrolle des vom Plasmid pKK223-3 kodierten tac-Promotors.

Zur Isolation des panD-Gens aus dem Plasmid pCR®2.1panD wurde die isolierte Plasmid DNA mit dem Enzym EcoRI gespalten, der Spaltungsansatz im 0,8 %igen Agarosegel aufgetrennt (30 Minuten, 10V/cm) und das 400 bp panD-Fragment mit Hilfe des GLASSMAX™ Kits isoliert. Das isolierte panD-Fragment wurde mit dem ebenfalls EcoRI gespaltenen Plasmid pKK223-3 mittels T4 DNA Ligase ligiert und der E. coli Stamm DH5αmcr mit dem Ligationsansatz elektroporiert. Die Selektion auf Plasmid tragende Zellen erfolgte durch Ausplattieren des Elektroporationsansatzes auf LB Agar, welcher mit 100µg/ml Ampicillin versetzt war und anschließender Inkubation für 24 Stunden bei 37°C. Das gesuchte Plasmid konnte nach DNA-Isolation und Kontrollspaltung, bei anschließender Gelelektrophorese im 0,8 %igen Agarosegel (30 Minuten, 10V/cm), mit den Enzymen EcoRI, EcoRV und HincII in einem Klon identifiziert werden und wurde mit pFE50 bezeichnet. Die Kodierregion des panD-Gens steht im Plasmid pFE50 unter der Kontrolle des vom Plasmid pKK223-3 kodierten tac-Promotors.

Das panC-Gen wurde mittels einer HindIII-SmaI (Pharmacia Biotech (Freiburg, Deutschland), Produktbeschreibung SmaI, Code no. 27-0942-01) -Spaltung aus dem Plasmid pFE60 isoliert, dazu wurde der Spaltungsansatz im 0,8 %igen Agarosegel aufgetrennt (30 Minuten, 10V/cm) und das 850 bp panC-Fragment mit Hilfe des GLASSMAX™ Kits isoliert. Das isolierte panC-Fragment wurde mit dem ebenfalls HindIII und SmaI gespaltenen Plasmid pFE50 mittels T4 DNA Ligase ligiert und der E. coli Stamm DH5αmcr mit dem Ligationsansatz elektroporiert. Die Selektion auf Plasmid tragende Zellen erfolgte durch Ausplattieren des Elektroporationsansatzes auf LB Agar, welcher mit 100µg/ml Ampicillin versetzt war und anschließender Inkubation für 24 Stunden bei 37°C. Das gesuchte Plasmid konnte nach DNA-Isolation und Kontrollspaltung, bei anschließender Gelelektrophorese im 0,8 %igen Agarosegel (30 Minuten, 10V/cm), mit den Enzymen EcoRI, EcoRV, SmaI, HindIII und HincII in einem Klon identifiziert werden und wurde mit pFE52 bezeichnet. Die Kodierregion des panD-Gens und des panC-Gens stehen im Plasmid pFE52 unter der Kontrolle des vom Plasmid pKK223-3 kodierten tac-Promotors und bilden ein Operon.

In die auf den tac-Promotor folgende EcoRI-Schnittstelle des Plasmids pFE52 wurde das panB-Gen kloniert und das erhaltene Plasmid mit pFE70 bezeichnet. Das panB-Gen wurde dazu aus einem Agarosegel (30 Minuten, 10V/cm) isoliert, in welchen ein EcoRI-Restriktionsansatz des Plasmids pFE40 aufgetrennt wurde. Ligation erfolgte mit T4-DNA-Ligase. Nach Elektroporation des Ligationsansatzes in den Stamm SJ2 wurden die Transformanten auf MediumE-Agar, welcher mit 0,4% Glucose, 100µg/ml Thiamin und 100µg/ml Ampicillin versetzt war, selektioniert. DNA aus Ampicillin resistenten Kolonien wurde mit dem QIAprep Spin Plasmid Kit isoliert und der Erfolg der Klonierung mittels einer EcoRI-, EcoRV-, SmaI-, HindIII- und HincII-Spaltung und anschließender Auftrennung im 0,8 %igen Agarosegel (30 Minuten, 10V/cm) verifiziert. Die Kodierregion des panB-Gens, panD-Gens und des panC-Gens stehen im Plasmid pFE70 unter der Kontrolle des vom Plasmid pKK223-3 kodierten tac-Promotors und bilden ein Operon.

Das panE-Gen wurde mittels einer HindIII-SphI (Pharmacia Biotech (Freiburg, Deutschland), Produktbeschreibung SphI, Code no. 27-0951-01) -Spaltung aus dem Plasmid pFE65 isoliert, dazu wurde der Spaltungsansatz im 0,8 %igen Agarosegel aufgetrennt (30 Minuten, 10V/cm) und das panE-Fragment mit Hilfe des GLASSMAX™ Kits isoliert. Das isolierte panE-Fragment wurde mit dem ebenfalls HindIII und partiell SphI gespaltenen Plasmid pFE70 mittels T4 DNA Ligase ligiert und der Stamm FE5 mit dem Ligationsansatz elektroporiert. Die Selektion auf Plasmid tragende Zellen erfolgte durch Ausplattieren des Elektroporationsansatzes auf MedienE-Agar + Glucose (0,4%) + 50µg/ml Isoleucin + 50µg/ml Ketoisovalerat, welcher mit 100µg/ml Ampicillin versetzt war und anschließender Inkubation für 48 Stunden bei 37°C. Das gesuchte Plasmid konnte nach DNA-Isolation und Kontrollspaltung, bei anschließender Gelelektrophorese im 0,8 %igen Agarosegel (30 Minuten, 10V/cm), mit den Enzymen EcoRI, EcoRV, SphI, HindIII und HincII in einem Klon identifiziert werden und wurde mit pFE80 bezeichnet. Die Kodierregion des panB-Gens, panD-Gens, panC-Gens und des panE-Gens stehen im Plasmid pFE80 unter der Kontrolle des vom Plasmid pKK223-3 kodierten tac-Promotors und bilden ein Operon.

Das Plasmid pFE80 wurde in den Stamm E. coli K12 MG1655 elektroporiert und Transformanten auf LB Agar, welcher mit 100µg/ml Ampicillin versetzt war, und anschließender Inkubation für 24 Stunden bei 37°C selektioniert. Der erhaltene Stamm wurde als MG1655/pFE80 bezeichnet.

### Beispiel 6

### Überexpression des panE-Gens von E. coli zusammen mit panB, panC und panD von E. coli in einer Valin-resistenten Mutante von E. coli K12 MG1655

Der E. coli K12 Stamm MG1655 wurde auf MediumE-Agar, welcher mit 0,4% Glucose und 100 µg/ml Valin (Sigma (Deisenhofen, Deutschland),V0258) versetzt war, ausgestrichen. Nach 48 Stunden Inkubation bei 37°C konnte eine Kolonie isoliert werden. Dieser Stamm wurde mit FE6 bezeichnet. Das Plasmid pFE80 wurde in den Stamm FE6 elektroporiert und Transformanten auf LB Agar, welcher mit 100µg/ml Ampicillin versetzt war, und anschließender Inkubation für 24 Stunden bei 37°C selektioniert. Der erhaltene Stamm wurde als FE6/pFE80 bezeichnet.

### Beispiel 7

### Überexpression des panE-Gens von E. coli zusammen mit panB, panC und panD von E. coli in einer avtA::aadB-Mutante von E. coli K12 MG1655

Ausgehend von der Nukleotidsequenz für das avtA-Gen (EMBL-GenBank: Accession Nr. Y00490) in E. coli K12 MG1655 wurden PCR-Primer synthetisiert (MWG Biotech (Ebersberg, Deutschland)). Ein ca. 1,6 kbp großes DNA-Fragment konnte mit diesen Primern unter der Standard-PCR-Methode aus chromosomaler E. coli K12 MG1655 DNA amplifiziert werden. Die Größe wurde durch gelelektrophoretische Auftrennung (30 Minuten, 10V/cm) in einem 0,8 %igen Agarosegel bestimmt.

PCR-Primer für das avtA-Gen aus E. coli:

| | | |
|---|---|---|
| avtA1 | 5' - TGCTCTCTCTCAACGCCGAA | -3' |
| avtA2 | 5' - GAAGCCGCCAACCAGGATAA | -3' |

Das PCR-Produkt des avtA-Gens wurde in das Plasmid pCR®2.1 und in den E. coli Stamm TOP10F*'* transformiert (Invitrogen (Leek, Niederlande)). Der Klonierungserfolg wurde durch Spaltung der DNA des Plasmids pCR®2.1avtA mit den Restriktionsenzymen EcoRI und SmaI nachgewiesen. Dazu wurde die Plasmid DNA mittels des QIAprep Spin Plasmid Kits isoliert und nach Spaltung in einem 0,8 %igen Agarosegel aufgetrennt (30 Minuten, 10V/cm). In die SmaI-Schnittstelle des Plasmids pCR®2.1avtA wurde ein aadB-Gen kloniert und das erhaltene Plasmid mit pFE23 bezeichnet. Das aadB-Gen wurde dazu aus einem Agarosegel (30 Minuten, 10V/cm) isoliert, in welchen ein SmaI-Restriktionsansatz des Plasmids pHP45Ω (EMBL-GenBank: Accession Nr. K02163) aufgetrennt wurde. Ligation erfolgte mit T4-DNA-Ligase. Nach Elektroporation des Ligationsansatzes in den Stamm DH5αmcr wurden die Transformanten auf PA-Agar, welcher mit 20 µg/ml Streptomycin (Sigma (Deisenhofen, Deutschland), Code no. S6501) versetzt war, selektioniert. DNA aus Streptomycin resistenten Kolonien wurde mit dem QIAprep Spin Plasmid Kit isoliert und der Erfolg der Klonierung mittels einer EcoRI- und SphI-Spaltung und anschließender Auftrennung im 0,8 %igen Agarosegel (30 Minuten, 10V/cm) verifiziert.

Das avtA::aadB-Fragment wurde aus dem Plasmid pFE23 mittels EcoRI-Restriktion gespalten, im 0,8 %igen Agarosegel (30 Minuten, 10V/cm) aufgetrennt und mit dem GLASSMAX™ Kit isoliert. Das Fragment wurde mit dem partiell EcoRI gespaltenem Plasmid pMAK705 mittels T4-DNA-Ligase ligiert, der Ligationsansatz in den Stamm DH5αmcr elektroporiert und Transformanten durch Inkubation auf LB-Agar + 20µg/ml Streptomycin + 25µg/ml Chloramphenicol für 24 Stunden bei 30°C selektioniert. DNA aus Streptomycin und Chloramphenicol resistenten Kolonien wurde mit dem QIAprep Spin Plasmid Kit isoliert und der Erfolg der Klonierung mittels einer SphI- und EcoRI-Spaltung im 0,8 %igen Agarosegel (30 Minuten, 10V/cm) nachgewiesen. Das konstruierte Plasmid wurde mit pFE24 bezeichnet.

Mit Hilfe des Plasmids pFE24 wurde in dem Stamm E. coli K12 MG1655 das chromosomale avtA-Gen gegen das avtA::aadB-Allel ausgetauscht. Für den Genaustausch wurde eine modifizierte Methode nach Hamilton et al. verwendet. Das Plasmid pFE24 wurde in den E. coli K12 MG1655 Stamm elektroporiert, anschließend wurden die Transformanten zur Selektion auf Cointegrate auf LB-Chloramphenicol-Agar bei 42°C für 24 Stunden inkubiert. Die erhaltenen Kolonien wurden zur Vereinzelung wiederum auf dem gleichen Medium ausgestrichenund bei 42°C für 24 Stunden inkubiert. Zur Desintegration des Plasmids wurden Einzelkolonien in 5ml LB-Flüssigmedium bei 42°C für 24 Stunden inkubiert, und anschließend eine Verdünnungsreihe des Flüssigmediums auf LB-Chloramphenicol-Agar ausplattiert. Diese Verdünnungsreihe wurde bei 30°C für 24 Stunden inkubiert. Zur Kurierung vom Plasmid wurden aus der Verdünnungsreihe erhaltene Einzelkolonien in 3 aufeinanderfolgenden Einzelkolonieausstrichen auf LB-Agar bei 42°C für jeweils 24 Stunden angezogen.
Zur Kontrolle des Phänotyps wurden die erhaltenen Einzelkolonien parallel auf Agarplatten mit LB- Medium + 20µg/ml Streptomycin und LB-Medium + 25µg/ml Chloramphenicol angeimpft. Diese Medien wurden für 48 Stunden bei 37°C inkubiert. Unter 250 getesteten Einzelkolonien befand sich eine deren Phänotyp den Austausch des chromosomalen avtA-Gens durch das avtA::aadB-Fragment anzeigte. Dieser Stamm wurde mit FE7 bezeichnet.

Das Plasmid pFE80 wurde in den Stamm FE7 elektroporiert und Transformanten auf LB Agar, welcher mit 100µg/ml Ampicillin versetzt war, und anschließender Inkubation für 24 Stunden bei 37°C selektioniert. Der erhaltene Stamm wurde als FE7/pFE80 bezeichnet.

### Beispiel 8

### Bestimmung der Ketopantoatreduktase-Aktivität in verschiedenen Stämmen von Escherichia coli K12

Die spezifische Ketopantoatreduktase Aktivität wurde nach der bei Shimizu et al. (Journal of Biological Chemistry 263: 12077-12084 (1988)) beschriebenen Methode bestimmt. Hierzu wurden, mittels eines Hybaid RiboLyser (Heidelberg, Deutschland) und des RiboLyser Kit Blue, Zellextrakte der einzelnen Stämme gewonnen. Die Ketopantoatreduktase Aktivität der Extrakte wurde anhand des NADPH Verbrauchs bei Zugabe von Ketopantoat bestimmt. Für den Stamm E. coli K12 MG1655 wurde eine spezifische Ketopantoatreduktase Aktivität von 6,5 mU/mg, für den Stamm E. coli K12 MG1655/pFE65 eine von 22,0 mU/mg bestimmt. Im Falle von Stamm FE5 war keine Aktivität messbar.

### Beispiel 9

### Bildung von Pantothenat durch verschiedene Stämme von Escherichia coli K12

Die Bildung von Pantothenat durch die Stämme MG1655, MG1655/pFE32, MG1655/pFE65, MG1655/pFE80, FE6/pFE80 und FE7/pFE80 wurde in batch - Kultur geprüft. Als Kulturmedium wurde das von Vogel (Journal of Biological Chemistry 1956, 218: 97-106) beschriebene MediumE mit Glucose (0,4%) als Kohlenstoffquelle verwendet. Die Zusammensetzung des verwendeten Mediums ist in Tabelle 2 dargestellt.

**Tabelle 2**

| Verbindung | Konzentration |
|---|---|
| MnSO₄*7H₂O | 0,2 g/l |
| Zitronensäure-Monohydrat | 2,0 g/l |
| K₂HPO₄ | 10,0 g/l |
| NaNH₄HPO₄*H₂O | 3,5 g/l |

250 ml Erlenmeyerkolben wurden mit 25 ml des angegebenen Nährmediums befüllt und der Ansatz beimpft. Nach einer Bebrütungszeit von 48 Stunden bei 37°C wurden die optische Dichte und die Pantothenat-Konzentration bestimmt. Für die Bestimmung der Zelldichte wurde die optischen Dichte mit einem Novaspec II Photometer-Photometer der Firma Pharmacia (Freiburg, Deutschland)bei einer Meßwellenlänge von 580 nm eingesetzt. Der Pantothenat-Gehalt wurde im steril filtriertem Kulturüberstand bestimmt. Die Pantothenatbestimmung (als Calciumsalz) erfolgte mit Hilfe des Stammes Lactobacillus plantarum ATCC® 8014 nach Angaben des Handbuchs "DIFCO MANUAL" der Firma DIFCO (Michigan, USA;, 10th Edition, 1100-1102 (1984)). Das Ergebnis ist in Tabelle 3 zusammengefasst.

**Tabelle 3**

| Stamm | Konzentration [µg/ml] | Zelldichte [oD₅₈₀] | Produktivität [µg/ml/oD₅₈₀] |
|---|---|---|---|
| MG1655 | 0,51 | 2,8 | 0,18 |
| MG1655/pFE32 | 1,7 | 2,8 | 0,60 |
| MG1655/pFE65 | 4,6 | 2,9 | 1,6 |
| M61655/pFE80 | 14,0 | 2,9 | 4,8 |
| FE6/pFE80 | 35,7 | 3,2 | 11,2 |
| FE7/pFE80 | 41,7 | 3,0 | 13,9 |

### Beispiel 10

### Bildung von Pantothenat durch verschiedene Stämme von Escherichia coli K12 in Gegenwart von Ketopantoat

Die Bildung von Pantothenat durch die Stämme MG1655, MG1655/pFE32, MG1655/pFE65 bei zugesetztem Ketopantoat wurde in batch - Kultur geprüft. Hierzu wurde das in Beispiel 8 beschriebene Medium mit 50 µg/ml Ketopantoat supplementiert. Die übrigen Versuchsbedingungen sind wie in Beispiel 8 angegeben. Das Ergebnis ist in Tabelle 4 zusammengefasst.

**Tabelle 4**

| Stamm | Konzentration [µg/ml] | Zelldichte [oD₅₈₀] | Produktivität [µg/ml/oD₅₈₀] |
|---|---|---|---|
| MG1655 | 6,2 | 2,9 | 2,1 |
| MG1655/pFE32 | 9,0 | 2,9 | 3,1 |
| MG1655/pFE65 | 12,6 | 2,9 | 4,3 |

### Beispiel 11

### Isolierung des ilvC-Gens von Corynebacterium glutamicum ATCC13032

Chromosomale DNA aus C. glutamicum ATCC 13032 wurde wie bei Tauch et al. (Plasmid, 33:168-179, 1995) beschrieben, isoliert und mit der Restriktionsenzym Sau3A (Pharmacia Biotech (Freiburg, Deutschland), Produktbeschreibung Sau3A, Code no. 27-0913-02) partiell gespalten. DNA-Fragmente in einem Größenbereich von 7-9 kb wurden mit Hilfe des "Nucleotrap Extraction Kit for Nucleic Acids" (Macherey und Nagel, Düren, Deutschland; Cat. No. 740584) isoliert und in die dephosphorylierte BamHI-Schnittstelle des Vektors pUC19 (Viera et al., 1982, Gene, 19:259-268; MBI Fermentas, Litauen) ligiert. Die Ligation wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5aMCR (Grant, 1990, Proceedings of the National Academy of Sciences U.S.A., 87:4645-4649) elektroporiert (Tauch, 1994, FEMS Microbiological Letters, 123:343-348) und auf LB-Agar (Lennox, 1955, Virology, 1:190) + 100 mg/ml Ampicillin ausplattiert. Nach Inkubation für 24 h bei 37°C konnte die C. glutamicum Genbank durch Reisolierung der Plasmid-DNA nach der "Alkalischen-Lyse-Methode" von Birnboim und Doly (1997, Nucleic Acids Research, 7: 1513-1523) aus den Transformanden gewonnen werden. Mit dieser Genbank wurden kompetente Zellen des E. coli Stamms FE5, welcher Mutationen im panE und ilvC Gen trägt, elektroporiert. Der Elektroporationsansatz wurde im Anschluß an die Regenerationsphase (Tauch et.al., 1994, FEMS Microbiological Letters, 123:343-347) zweimal mit Medium E (Vogel and Bonner, 1956, Journal of Biolgical Chemistry, 218:97-106) gewaschen. Die Selektion der Transformanten erfolgte durch Ausplattieren auf Medium E-Agar + Glucose (0,4%) + 100 µg/ml Ampicillin + 50µg/ml Isoleucin + 50µg/ml Ketoisovalerat. Aus 4 erhaltenen Kolonien wurde mit Hilfe des QIAprep Spin Plasmid Kits die Plasmid DNA isoliert. Durch eine XbaI-Spaltung der Plasmid DNA und anschließender Auftrennung im 0,8 %igen Agarosegel (30 Minuten, 10V/cm) wurde gezeigt, daß es sich bei den Plasmiden um pUC19 Vektoren mit ca. 6,5 kbp großen Inserts handelte. Die Sequenzierung der Inserts mit anschließenden Homologievergleichen mit Hilfe des BLAST-Programms (Altschul, Journal of Molecular Biology 1990, 215: 403-410) ergab, daß die Inserts in allen Fällen ein vollständiges ilvC-Gen aus C. glutamicum enthielten (EMBL-GenBank: Accession Nr. L09232). Eines dieser Plasmide wurde mit pFE90 bezeichnet.

### Beispiel 12

### Expression des ilvC-Gens von Corynebacterium glutamicum ATCC13032 in Corynebacterium glutamicum ATCC13032

Zur Expression des ilvC-Gens aus C. glutamicum in C. glutamicum ATCC13032, wurde das Plasmid pECm3 verwendet. Das Plasmid pECm3 ist ein Derivat des Plasmids pECm2 (Tauch, 1994, FEMS Microbiological Letters, 123:343-348), dessen Kanamycin-Resistenzgen durch eine BglII- (Pharmacia Biotech (Freiburg, Deutschland), Produktbeschreibung BglII, Code no. 27-0946-02)und BamHI-Restriktion mit anschließender Religation entfernt wurde. Die Plasmide pECm2 sowie pECm3 sind in der Lage, sowohl in E. coli als auch in C. glutamicum zu replizieren. Zur Isolation des ilvC-Gens aus dem Plasmid pFE90 (Beispiel 11) wurde die isolierte Plasmid DNA mit dem Enzym XbaI (Pharmacia Biotech (Freiburg, Deutschland), Produktbeschreibung XbaI, Code no. 27-0948-01)gespalten, der Spaltungsansatz im 0,8 %igen Agarosegel aufgetrennt (30 Minuten, 10V/cm) und das 6,5 kbp ilvC-Fragment mit Hilfe des GLASSMAX™ Kits isoliert. Das isolierte ilvC-Fragment wurde mit dem ebenfalls XbaI gespaltenen Plasmid pECm3 mittels T4 DNA Ligase ligiert und der E. coli Stamm FE5 mit dem Ligationsansatz elektroporiert. Die Selektion auf Plasmid tragende Zellen erfolgte durch Ausplattieren des Elektroporationsansatzes auf LB Agar, welcher mit 50µg/ml Chloramphenicol versetzt war und anschließender Inkubation für 24 Stunden bei 37°C. Das gesuchte Plasmid konnte nach DNA-Isolation und Kontrollspaltung, bei anschließender Gelelektrophorese im 0,8 %igen Agarosegel (30 Minuten, 10V/cm), mit dem Enzym XbaI in einem Klon identifiziert werden und wurde mit pFE91 bezeichnet.

Das Plasmid pFE91 wurde in den Stamm C. glutamicum ATCC13032 elektroporiert und Transformanten auf LB Agar, welcher mit 7,5µg/ml Chloramphenicol versetzt war, und anschließender Inkubation für 48 Stunden bei 30°C selektioniert. Der erhaltene Stamm wurde als C. glutamicum ATCC13032/pFE91 bezeichnet.

### Beispiel 13

### Bildung von Pantothenat durch Corynebacterium glutamicum ATCC13032

Die Bildung von Pantothenat durch den C. glutamicum Stamm ATCC13032/pFE91 wurde in Medium CGXII (Keilhauer et al., 1993, Journal of Bacteriology, 175:5595-5603) mit 10 mg/ml Chloramphenicol, im Folgenden als C. glutamicum-Testmedium bezeichnet, geprüft. Dieses Medium ist in Tabelle 5 dargestellt. Je 50 ml frisch angesetztes C. glutamicum-Testmedium wurden mit einer 16 Stunden alten Kultur (C. glutamicum-Testmedium, 30°C, 150 Upm) mit einer o.D.₅₈₀ von 0,1 angeimpft. Nach 48stündiger Inkubation bei 30 C und 150 U/min wurden die Zellen durch 10minütige Zentrifugation bei 5000 x g entfernt, der Überstand wurde sterilfiltriert und die Pantothenat-Konzentration bestimmt. Die Zelldichte wurde wie in Beispiel 9 beschrieben bestimmt.

Die Pantothenatbestimmung (als Calciumsalz) erfolgte mit Hilfe des Stammes Lactobacillus plantarum ATCC® 8014 nach Angaben des Handbuchs "DIFCO MANUAL" der Firma DIFCO (Michigan, USA;, 10^{th} Edition, 1100-1102 (1984)). Das Ergebnis ist in Tabelle 6 dargestellt.

**Tabelle 5**

| Substanz | Menge pro Liter | Bemerkung |
|---|---|---|
| (NH₄)₂ SO₂ | 20 g | |
| Harnstoff | 5 g | |
| KH₂KO₄ | 1 g | |
| K₂HPO₄ | 1 g | |
| MgSO₄ * 7 H₂O | 0.25 g | |
| MOPS | 42 g | |
| CaCl₂ | 10 mg | |
| FeSO₄ * 7 H₂O | 10 mg | |
| MnSO₄ * H₂O | 10 mg | |
| ZnSO₄ * 7 H₂O | 1 mg | |
| CuSO₄ | 0.2 mg | |
| NiCl₂ * 6 H₂O | 0.02 mg | |
| Biotin | 0.5 mg | |
| Glukose | 40 g | separat autoklavieren |
| Protocatechusäure | 0.03 mg | sterilfiltrieren |

**Tabelle 6**

| Stamm | Konzentration [µg/ml] | Zelldichte [oD₅₈₀] | Produktivität [µg/ml/oD₅₈₀] |
|---|---|---|---|
| ATCC13032 | 0,2 | 20 | 0,010 |
| ATCC13032/pFE91 | 0,3 | 20 | 0,015 |

### Beispiel 14:

### Expression des panE-Gens von Saccharomyces cerevisiae

### 1. Amplifikation des Leserasters YHR063c:

Ausgehend von der Nukleotidsequenz des Saccharomyces cerevisiae Leserasters YHR063c (Accession Nr. U00061 des National Center for Biotechnology, Bethesda, MD, USA) wurden die nachstehenden PCR-Primer synthetisiert (MWG-Biotech, Ebersberg, Deutschland). Anfang bzw. Ende des Leserasters sind durch einen Punkt (.) gekennzeichnet:
- oJD539 (5' EcoRI-NotI START): 
5'- GCG CGA ATT CAG ATC CGC GGC CGC AAA GAG GAG AAA TTA
ACT.ATG ACT GCA CCA CAC AGA AG -3'
- oJD540 (3' SpeI-PstI STOP):
   5'- CGC GAC TAG TCT GCA G.TC AGT CCT TTC TCC AGT CAC-3'

Als Template diente genomische DNA des S. cerevisiae Stammes JD242, die nach der Methode von C. Guthrie und G.R. Fink (Guide to yeast genetics and molecular biologgy, Methods in Enzymology, Vol. 194, Academic Press, San Diego, CA, 1991) isoliert wurde. Dieser Stamm ist eine haploide Segregante des diploiden Stammes SC288C (Winston et al., Yeast 11, 53ff. (1995)), dessen Genom sequenziert wurde (Goffeau et al., Science 274, pp. 546, (1996)). Die Tetradenanalyse erfolgte nach der Methode von C. Guthrie und G.R. Fink (Guide to yeast genetics ans molecular biology, Methods in Enzymology, Vol. 194, Academic Press, San Diego, CA, 1991). Der Stamm JD242 ist auxotroph für Leucin (leu2Δ1 Allel) und Uracil (ura3-52 Allel). Ein etwa 1,2 kB großes DNA-Fragment konnte unter Verwendung des "High Fidelity Expand Polymerase" Kits der Firma Roche (Mannheim) durch 28 PCR-Zyklen unter den vom Hersteller angegebenen Bedingungen amplifiziert werden. Die Größe wurde durch elektrophoretische Auftrennung in einem 0,8%igen Agarosegel bestimmt.

### 2. Konstruktion von pJD-YHR063c:

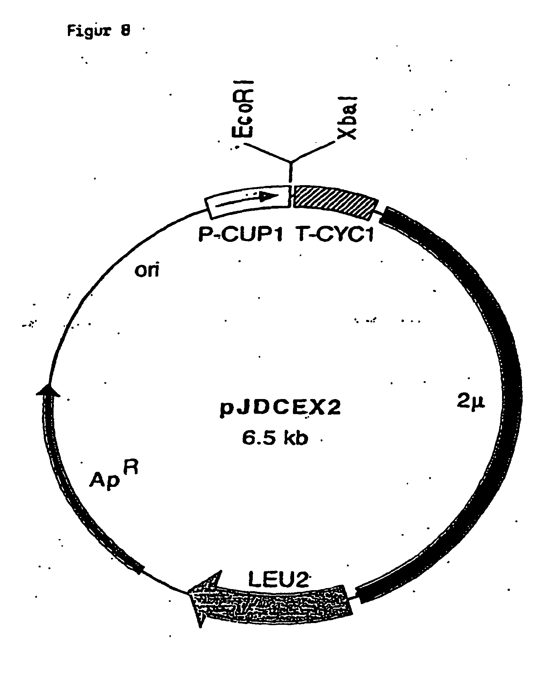

Zur Expression des YHR063c Leserasters in S. cerevisiae wurde das PCR-Amplifikat in den E. coli - S. cerevisiae Pendelvektor pJDCEX2 eingebaut (Abbildung 8 und Dohmen et al., 1995, Journal of Biological Chemistry 270, 18099-18109)

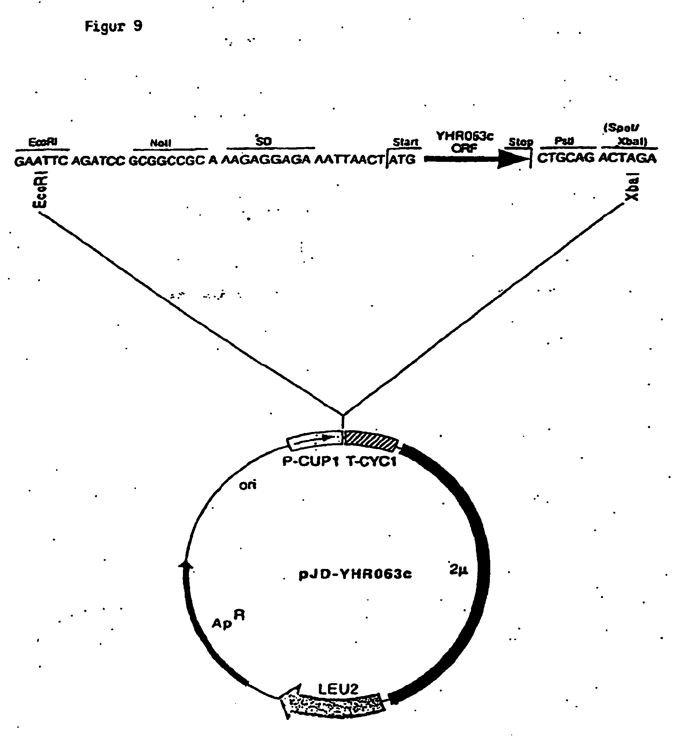

Das PCR-Produkt wurde zunächst mit EcoRI und SpeI (AGS, Heidelberg, Deutschland) restringiert. Anschließend wurde es mit pJDCEX2-DNA, welche mit EcoRI und XbaI (AGS, Heidelberg, Deutschland) behandelt worden war, gemischt und mit T4-DNA Ligase (Roche, Mannheim, Deutschland) ligiert. Der Ligationsansatz wurde in den E. coli Stamm XL1-Blue (Bullock et al., 1987, Biotechniques 5, 376) transformiert. Tranformanten wurde durch Selektion auf LB-Agar, welcher 150 µg/ml Ampicillin (Sigma, Deisenhofen, Deutschland) enthielt, erhalten. Die Plasmid-DNA aus den Ampicillin resistenten Klonen wurde durch alkalische Lyse präpariert (Sambrook et al.: Molecular Cloning, A Laboratory Manual, Cold Spring Harbor Laboratory Press, 1989). Die isolierte Plasmid-DNA wurde dann durch Restriktion mit NotI und PstI und anschließender Auftrennung im 0,8%igen Agarosegel untersucht. Das Plasmid mit der gewünschten Struktur erhielt den Namen pJD-YHR063c (Abbildung 9). Die Sequenz des in pJD-YHR063c klonierten PCR-Produktes wurde durch Sequenzierung mit den Oligonukleotiden oJD105 und oJD106 verifiziert.
- oJD105 (T-CYC1):
   5'- GAAGTCATCGAAATAG-3'
- oJD106 (P-CUP1):
   5'-TCGTTTCTGTCTTTTTC-3'

### 3. Konstruktion von pKK-YHR063c:

Zur Expression des YHR063c-Leserasters in E. coli wurde das Plasmid pKK223-3 (Brosius and Holy, Proceedings of the National Academy of Science USA 81, 6929 (1984)) verwendet. Hierzu wurde das Plasmid pJD-YHR063c zunächst mit EcoRI und PstI (AGS, Heidelberg, Deutschland) restringiert. Das etwa 1,2 kB große YHR063c-Fragment wurde nach elektrophoretischer Auftrennung in einem 0,8%igen Agarosegel aus diesem ausgeschnitten und die DNA mit dem QiaexII Gel Extraction Kit (Qiagen, Hilden, Deutschland) isoliert. Anschließend wurde sie in das Plasmid pKK223-3, welches mit EcoRI und PstI geöffnet worden war, mit T4-DNA Ligase (Roche, Mannheim, Deutschland) ligiert. Der Ligationsansatz wurde in den E. coli Stamm XL1-Blue (Stratagene, LaJolla, CA, USA) transformiert. Tranformanten wurde durch Selektion auf LB-Medium, welches 150 µg/ml Ampicillin (Sigma, Deisenhofen, Deutschland) enthielt, erhalten. Die Plasmid-DNA aus den Ampicillin resistenten Klonen wurde durch alkalische Lyse präpariert (Sambrook et al.: Molecular Cloning, A Laboratory Manual, Cold Spring Harbor Laboratory Press, 1989). Der Klonierungserfolg wurde durch Restriktion mit EcoRI und PstI und anschließender Auftrennung im 0,8%igen Agarosegel überprüft. Das Plasmid mit der gewünschten Struktur erhielt den Namen pKK-YHR063c.

### Beispiel 15:

### Komplementation der E. coli Mutante FE5

Zur Analyse der panE-Funktion des YHR063c Leserasters aus S. cerevisiae wurde untersucht, ob die Expression dieses Leserasters die Pantothensäure-Bedürftigkeit des E. coli Stammes FE5 (Beispiel 1)komplementieren kann. Dieser Stamm ist in den Genloci panE und ilvC mutiert. Hierzu wurde zunächst der Stamm FE5 mit Plasmid pKK-YHR063c transformiert.

Anschließend wurde das Wachstum des Stammes FE5/pKK-YHR063c auf M9-Minimalagar (Sambrook et al.: Molecular Cloning, A Laboratory Manual, Cold Spring Harbor Laboratory Press, 1989), der mit 50 µg/ml Ketoisovalerat (Kiv) und 50 µg/ml Isoleucin (Ile) supplementiert worden war, in Abhängigkeit von der Zugabe von Pantothenat (50 µg/ml) untersucht. Als negative Kontrolle diente der Stamm FE5/pKK223-3 und als positive Kontrolle der Stamm FE4/pFE65 (Beispiel 4). Tabelle 7 zeigt das Ergebnis des Versuches: Das in Plasmid pKK-YHR063c enthaltene S. cerevisiae Leseraster YHR063c komplementiert die panE-ilvC Doppelmutation des E. coli Stammes FE5. Das Leseraster YHR063c hat die Funktion eines panE-Gens.

**Tabelle 7**

| Stamm | M9 + Kiv + Ile mit Pantothenat | M9 + Kiv + Ile ohne Pantothenat |
|---|---|---|
| FE5/pFE65 | Wachstum | Wachstum |
| FE5/pKK223-3 | Wachstum | kein Wachstum |
| FE5/pKK-YHR063c | Wachstum | Wachstum |

### Beispiel 16

### Bestimmung der Ketopantoatreduktase-Aktivität in verschiedenen Stämmen von Saccharomyces cerevisiae

Der S. cerevisiae Stamm JD242 (Siehe Beispiel 14) wurde mit den Plasmiden pJDCEX2 und pJD-YHR063c nach der Methode von Dohmen et al. transformiert (Dohmen et al., Yeast 7, 691(1991)). Die Selektion auf Transformanten erfolgte auf leucinfreiem Minimalmedium mit 1,8% Agar(siehe Tab. 8a,b).

Als Nährmedium wurde eine Pantotothensäure - freie Variante des im Difco-Manual (Michigan, USA;, 10^{th} Edition, 1100-1102 (1984)) beschriebenen Yeast Nitrogen Base-Minimalmediums (YNB) verwendet. Es enthielt zusätzlich Glucose (2%), Uracil (40 pg/ml), CuSO₄ (150 µM) zur Induktion des P*_{CUP1}*-Promotors von pJDCEX2 und pJD-YHR-063c, -Leu Drop-Out Supplement der Firma CLONTECH (Heidelberg, Deutschland, Cat. no. 8605-1) (650 pg/ml) und die Supplemente Ketopantoat (100 µg/ml) und β-Alanin (100 µg/ml). Die Zusammensetzung des verwendeten Mediums ist in Tabelle 8a und b dargestellt.Tabelle 8a:

**Tabelle 8a**

| Verbindung | Menge pro Liter |
|---|---|
| (NH₄)₂ SO₂ | 5 g |
| KH₂PO₄ | 1 g |
| MgSO₄ * 7 H₂O | 0,5 g |
| NaCl | 0,1 g |
| CaCl₂ | 0,1 g |
| H₃BO₃ | 500 µg |
| CuSO₄ | 40 µg |
| KI | 100 µg |
| FeCl₃ * 6 H₂O | 200 µg |
| MnSO₄ * H₂O | 400 µg |
| Na₂MoO₄ * 2 H₂O | 400 µg |
| ZnSO₄ * 7 H₂O | 200 µg |
| Biotin | 2 µg |
| Folsäure | 2 µg |
| Inositol | 2 mg |
| Niacin | 400 µg |
| p-Aminobenzoesäure | 200 µg |
| Pyridoxin Hydrochlorid | 400 µg |
| Riboflavin | 200 µg |
| Thiamin Hydrochlorid | 400 µg |

**Tabelle 8b**

| Zusätze | Menge pro Liter |
|---|---|
| Glucose | 20 g |
| Uracil | 40 mg |
| CuSO₄ | 24 mg |
| -Leu DO Supplement | 650 mg |
| Ketopantoat | 100 mg |
| β-Alanin | 100 mg |

250 ml Erlenmeyerkolben wurden mit 50 ml des angegebenen Nährmediums befüllt, der Ansatz mit Hilfe einer Impföse mit einer Einzelkolonie von einer Agarplatte beimpft (siehe Tab. 8a,b) und bei 30 C und 175 U/min 72 Stunden inkubiert. Mit dieser Vorkultur wurden 50 ml des gleichen Nährmedium in einem 250 ml Erlenmeyerkolben so mit der Vorkultur beimpft, das die optischen Dichte (580 nm) 0,5 betrug. Nach einer 24 stündigen Bebrütungszeit bei 30°C und 175 U/min wurden die optische Dichte mit einem Novaspec II Photometer der Firma Pharmacia (Freiburg, Deutschland) bei einer Meßwellenlänge von 580 nm gemessen. Sie betrug für beide Kulturen 4,0. Die spezifische Ketopantoatreduktase Aktivität der S. cerevisiae Stämme JD242/pJDCEX2 und JD242/pJD-YHR063c wurde nach der bei Shimizu et al. (Journal of Biological Chemistry 263: 12077-12084 (1988)) beschriebenen Methode bestimmt.

Hierzu wurden, mittels eines Hybaid RiboLyser (Heidelberg, Deutschland) und des RiboLyser Kit Red, Zellextrakte der einzelnen Stämme gewonnen. Die Ketopantoatreduktase Aktivität der Extrakte wurde anhand des NADPH Verbrauchs bei Zugabe von Ketopantoat bestimmt. Der Proteingehalt wurde nach der Methode von Bradfort ermittelt (Bradford, Analytical Biochemistry 72, 248ff.(1976)). Für den Kontrollstamm JD242/pJDCEX2 wurde eine spezifische Ketopantoatreduktase Aktivität von 3 mU/mg Protein und für der Stamm JD242/pJD-YHR063c eine spezifische Aktivität von 386 mU/mg Protein bestimmt.

### Beispiel 17

### Bildung von Pantothenat durch verschiedene Stämme von Saccharomyces cerevisiae

Die Bildung von Pantothenat durch die Stämme S. cerevisiae JD242/pJDCEX2 und JD242/pJD-YHR063c wurde in batch - Kultur geprüft.

250 ml Erlenmeyerkolben wurden mit 50 ml des in Tab. 8a,b angegebenen Nährmediums befüllt, der Ansatz mit Hilfe einer Impföse mit einer Einzelkolonie von einer Agarplatte beimpft (siehe Tabelle 8a,b) und bei 30°C und 175 U/min 72 Stunden inkubiert. Mit dieser Vorkultur wurden 50 ml des gleichen Nährmedium in einem 250 ml Erlenmeyerkolben so mit der Vorkultur beimpft, das die optischen Dichte (580 nm) 0,5 betrug. Nach einer 24 stündigen Bebrütungszeit bei 30°C und 175 U/min wurden die optische Dichte (580 nm) und die Pantothenat-Konzentration bestimmt. Für die Bestimmung der Zelldichte wurde die optischen Dichte mit einem Novaspec II Photometer der Firma Pharmacia (Freiburg, Deutschland) bei einer Meßwellenlänge von 580 nm gemessen. Der Pantothenat-Gehalt wurde im steril filtriertem Kulturüberstand bestimmt.

Die Pantothenatbestimmung (als Calciumsalz) erfolgte mit Hilfe des Stammes Lactobacillus plantarum ATCC® 8014 nach Angaben des Handbuchs "DIFCO MANUAL" der Firma DIFCO (Michigan, USA;, 10^{th} Edition, 1100-1102 (1984)). Das Ergebnis ist in Tabelle 9 zusammengefasst.

**Tabelle 9**

| S. cerevisiae Stamm | Konzentration [µg/ml] | Zelldichte [oD₅₈₀] | Produktivität [µg/ml/oD₅₈₀] |
|---|---|---|---|
| JD242/pJDCEX2 | 0,93 | 4,0 | 0,023 |
| JD242/pJD-YHR063c | 1,12 | 4,1 | 0,027 |

### Abbildungen

### Folgende Abbildungen sind beigefügt:

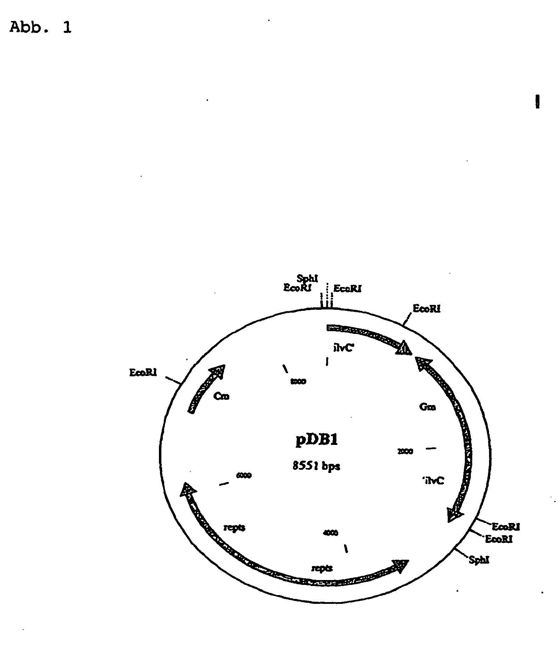

- Abbildung 1: Karte des Plasmids pDB1

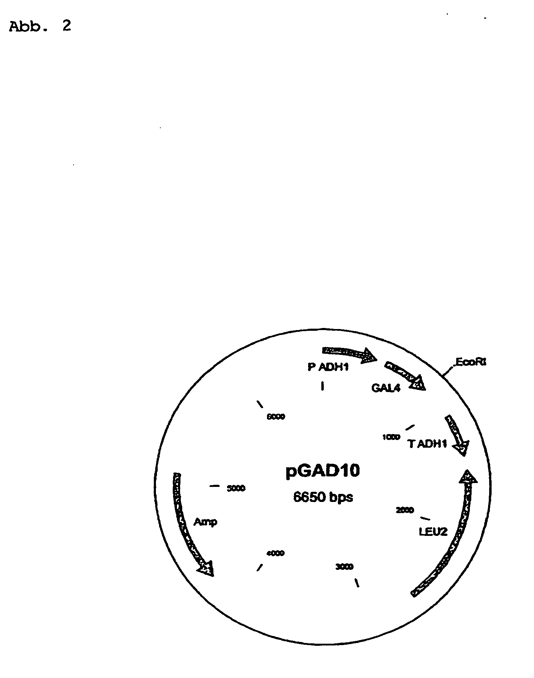

- Abbildung 2: Karte des Plasmids pGAD10

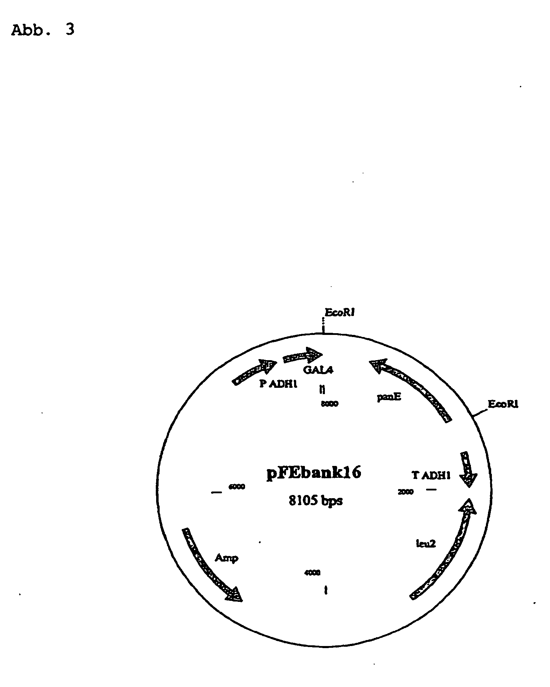

- Abbildung 3: Karte des Plasmids pFEbank16

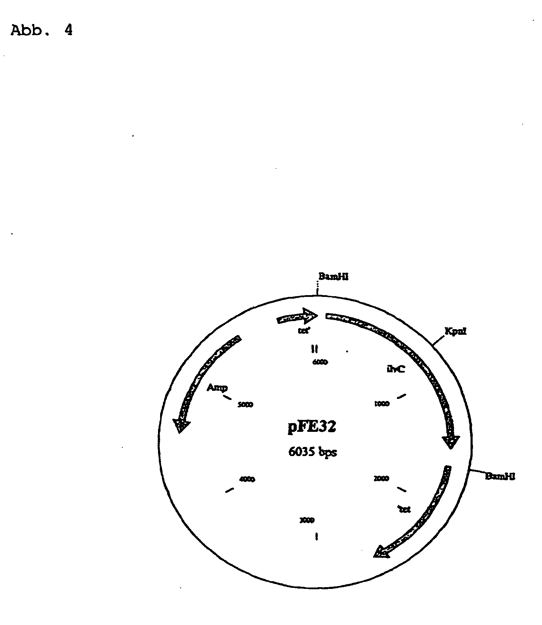

- Abbildung 4: Karte des Plasmids pFE32

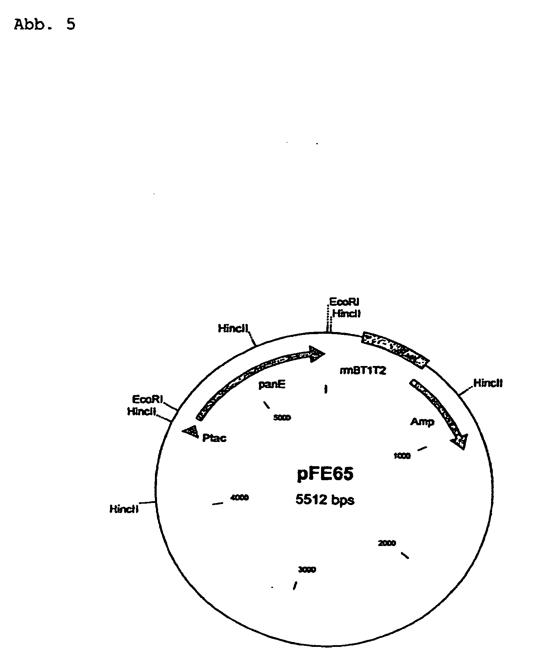

- Abbildung 5: Karte des Plasmids pFE65

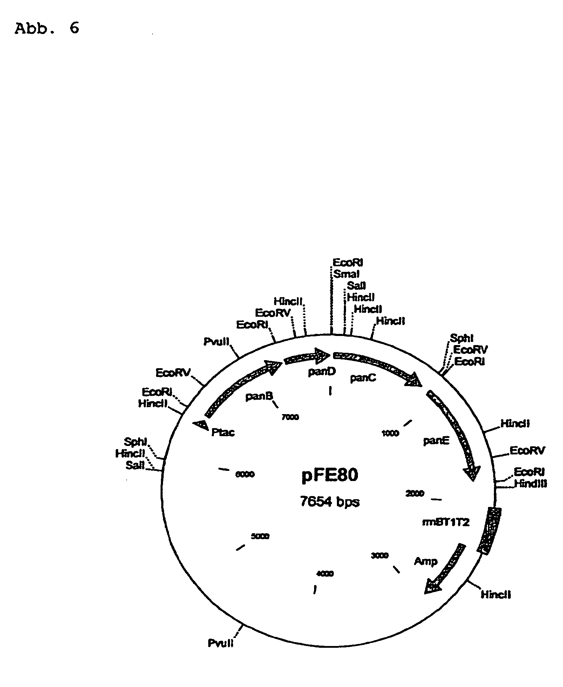

- Abbildung 6: Karte des Plasmids pFE80

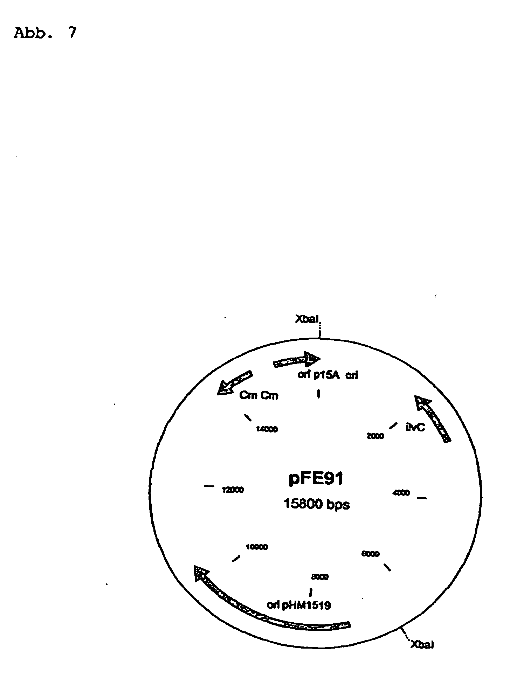

- Abbildung 7: Karte des Plasmids pFE91
- Abb.8: Karte des Plasmids pJDCEX2.
- Abb.9: Karte des Plasmids pJD-YHR063c.

Bei der Angabe der Basenpaarzahlen handelt es sich um ca.-Werte, die im Rahmen der Reproduzierbarkeit erhalten werden.

Die in den Abbildungen verwendeten Abkürzungen haben folgende Bedeutung:
- rrnBT1T2:: Transkriptions-Terminator des rrnB-Gens
- Ptac:: tac Promotor
- P AHD1:: Promotor des ADH1 Gens aus Saccharomyces cerevisiae
- T ADH1:: Terminator des ADH1 Gens aus Saccharomyces cerevisiae
- repts:: termosensitiver Replikationsursprung
- ilvC:: Kodierbereich des ilvC Gens
- ilvC':: 5'-Bereich des ilvC Gens
- 'ilvC:: 3'-Bereich des ilvC Gens
- panB:: Kodierbereich des panB Gens
- panC:: Kodierbereich des panC Gens
- panD:: Kodierbereich des panD Gens
- panE:: Kodierbereich des panE Gens
- Amp:: Resistenzgen für Ampicilin
- tet':: 5'-Bereich des tet Gens
- 'tet:: 3'-Bereich des tet Gens
- Cm:: Resistenzgen für Chloramphenicol
- Gm:: Resistenzgen für Gentamicin
- Ga14:: Regulator für Galactose induzierbare Gene aus Saccharomyces cerevisiae
- bps:: Basenpaare
- LEU2:: Beta-Isopropylmalat Dehydrogenase-Gen von Saccharomyces cerevisiae
- 2µ:: Sequenzen des endogenen 2µ Plasmides von Saccharomyces cerevisiae
- Ap^{R}:: Beta-Lactamase-Gen
- P-CUP1:: Promoter des Saccharomyces cerevisiae CUP1-Gens (Metallothionein)
- T-CYC1:: Terminator des CYC1-Gens (Cytochrom C) von Saccharomyces cerevisiae
- ORF:: open reading frame
- SD:: Shine-Dalgarno Sequenz
- EcoRI:: Schnittstelle des Restriktionsenzyms EcoRI
- EcoRV:: Schnittstelle des Restriktionsenzyms EcoRV
- HincII:: Schnittstelle des Restriktionsenzyms HincII
- HindIII:: Schnittstelle des Restriktionsenzyms HindIII
- KpnI:: Schnittstelle des Restriktionsenzyms KpnI
- SalI:: Schnittstelle des Restriktionsenzyms SalI
- SmaI:: Schnittstelle des Restriktionsenzyms SmaI
- SphI:: Schnittstelle des Restriktionsenzyms SphI
- PvuII:: Schnittstelle des Restriktionsenzyms PvuII
- NotI:: Schnittstelle des Restriktionsenzyms NotI aus Norcardia otitidis-cavarium
- SpeI:: Schnittstelle des Restriktionsenzyms SpeI aus Shpaerotilus spec.
- XbaI:: Schnittstelle des Restriktionsenzyms XbaI aus Xanthomonas badrii
- PstI:: Schnittstelle des Restriktionsenzyms PstI aus Providencia stuartii

## Patentansprüche

1. Verfahren zur Herstellung von O-Pantothensäure durch Fermentation diese Säure produzierender Mikroorgamsmen, **dadurch gekennzeichnet, dass** man Mikroorganismen einsetzt, in denen man für die Ketopantoatreduktase kodierende Nukleotidsequenzen (panE-Gen), überexprimiert.

2. Verfahren gemäß Anspruch 1, **dadurch gekennzeichnet, dass** man für die Ketopantoatreduktase kodierende Nukleotidsequenzen (panE-Gen) aus E. coli überexprimiert.

3. Verfahren gemäß Anspruch 2, **dadurch gekennzeichnet**, dann man das panE-Gen in den Mikroorganismen durch Plasmidvektoren exprimiert.

4. Verfahren gemäß den Ansprüchen 1 und 2, **dadurch gekennzeichnet, dass** man zur Erzielung der Überexpression als Promotor die lac-UV5-Mutation des lac-Promoters oder den tac Promoter einsetzt

5. Verfahren gemäß Anspruch 1,
**dadurch gekennzeichnet, dass** man Mikroorganismen einsetzt, in denen man das avtA-Gen ausschaltet.

6. Verfahren gemäß Anspruch 1, **dadurch gekennzeichnet, dass** man Mikroorganismen ersetzt, in denen man das ilvE-Gen ausschaltet.

7. Verfahren gemäß Anspruch 1, **dadurch gekennzeichnet, dass** man Mikroorganismen einsetzt, in denen man zusätzlich das ilvC-Gen überexprimiert

8. Verfahren gemäß Anspruch 1, **dadurch gekennzeichnet, dass** man Mikroorganismen der Gattungen Corynebactenum und E.coli einsetzt, in denen man zusätzlich das ilvC-Gen aus Corynebacterium glutamicum überexprimiert.

9. Verfahren gemäß Anspruch 1, **dadurch gekennzeichnet, dass** man zusätzlich eines oder mehrere der Gene überexprimiert ausgewählt aus der Gruppe:
9.1 das für die Ketopantoat-Hydroxymethyltransferase kodierende panB-Gen,
9.2 das für die Aspartat-Decarboxylase kodierende panD-Gen,
9.3 das für die Pantothenat-Synthetase kodierende panC-Gen.

10. Verfahren gemäß Anspruch 1, **dadurch gekennzeichnet, dass** man Mikroorganismen einsetzt, ausgewählt aus der Gruppe:
10.1 Gram-negative Bakterien,
10 2 Gram-positive Bakterien,
10 3 Pilze,
10.4 Hefen

11. Verfahren gemäß Anspruch 10, **dadurch gekennzeichnet, dass** es sich bei den Gram-negativen Mikroorganismen um Enterobacterlaceae handelt.

12. Verfahren gemäß Anspruch 11, **dadurch gekennzeichnet, dass** es sich bei den Mikroorganismen um die Art Escherichia coli handelt.

13. Verfahren gemäß Anspruch 10, **dadurch gekennzeichnet, dass** es sich bei den Gram-positiven Mikroorganismen um Mikrooganismen der Gattung Corynebacterium, handelt.

14. Verfahren gemäß Anspruch 1, **dadurch gekennzeichnet, dass** man die Mikroorganismen einsetzt, die eine L-Valin-Resistenz tragen.

15. Verfahren gemäß Anspruch 11, **dadurch gekennzeichnet, dass** man den Stamm FE7 einsetzt, der eine acta: aadB-Mutation im Chomosom trägt.

16. Verfahren gemäß Anspruch 10 oder 12, **dadurch gekennzeichnet, dass** man die Stämme Saccaromyces cerevislae JD242 oder E. coli FES einsetzt, in denen man die intrazelluläre Aktivität des Enzyms Ketopantoatreduktase durch Überexpression des Leserasters YHR063c aus der Art Saccharomyces cerevisiae erhöht.

17. Plasmidvektor pFE91, der das ilvC-Gen von Corynebacterium glutamicum trägt, **gekennzeichnet durch** die in Abbildung 7 wiedergegebene Restriktionskarte.

18. Plasmidvektor pJD-YHR063c, der das YHR063c Leseraster von Saccaromyces cerevisiae enthält, **gekennzeichnet durch** die in Abbildung 9 wiedergegebene Restriktionskarte.

19. Plasmidvektor pFE80, **gekennzeichnet durch** die in Abbildung 6 wiedergebende Restriktionskarte und hinterlegt in E.coli K12 Stamm MG 1655/pFE80 unter der Bezeichnung DSM 12414.

20. Plasmidvektor pFE65, **gekennzeichnet durch** die in Abbildung 5 wiedergegebende Restriktionskarte und hinterlegt in E.coli K12 Stamm MG1655/pFE65 unter der Bezeichnung DSM 12382.

21. Plasmidvektor pFE32, **gekennzeichnet durch** die in Abbildung 4 wiedergegebene Restriktionskarte und hinterlegt in E. coli K12 Stamm MG1655/pFE32 der Bezeichnung DSM 12413.

22. E.coli K12 Stamm FE6, der eine Valin-Resistenzmutation trägt, hinterlegt unter der Bezeichnung. DSM 12379.

23. E. coli K12 Stamm FE7, in dem das avtA-Gen gegen ein avtA: :aadB-Fragment ausgetauscht ist, hinterlegt unter der Bezeichnung DSM 12380.

24. Pantothensäure produzierende Mikroorganismen (Wirtszelle) der Gattungen E.coli oder Corynebacterium, die einen der Plasmidvektoren gemäß den Ansprüchen 19 bis 21 und gegebenenfalls eine oder mehrere Metabolit- und/oder Antimetabolit-Resistenz (en) enthalten. der das

25. Verfahren zur Herstellung von Pantothensäure,
**dadurch gekennzeichnet,**
**daß** man folgende Schritte durchführt:
a) Fermentation von Mikroorganismen, ausgewählt aus der Gruppe Gram-negative Bakterien, Gram-positive Bakterien, Pilze und Hefen, in denen man die für das Enzym Ketopantoatreduktase kodierenden Nukleotidsequenzen (panE-Gen) überexprimiert,
b) Anreicherung der Pantothensäure im Medium oder in den Zellen der Mikroorganismen,
c) isolieren der Pantothensäure.

26. Verfahren gemäß Anspruch 25, **dadurch gekennzeichnet**, das es sich bei den Gram-negativen Bakterien um Enterobacteriaceae handelt.

27. Verfahren gemäß Anspruch 26, **dadurch gekennzeichnet dass** es sich um Bakterien der Gattung Escherichia handelt.

28. Verfahren gemäß Anspruch 25, **dadurch gekennzeichnet, dass** es sich bei den Gram-positiven Bakterien um Mikroorganismen der Gattung Corynebacterium, handelt.

29. Verfahren gemäß Anspruch 28, **dadurch gekennzeichnet, dass** es sich bei den Bakterien der Gattung Corynebacterium um die Art Corynebacterium glutamicum. handelt.

30. Verfahren gemäß Anspruch 25, **dadurch gekennzeichnet, dass** in Stufe a) Ketopantoinsäure öder β-Alanin zugesetzt werden.

## Claims

1. Process for the production of D-pantothenic acid by fermentation of microorganisms producing this acid, **characterised in that** microorganisms are used in which nucleotide sequences (panE gene) coding for ketopantoate reductase are overexpressed.

2. Process according to claim 1, **characterised in that** nucleotide sequences (panE gene) from E. coli coding for ketopantoate reductase are overexpressed.

3. Process according to claim 2, **characterised in that** the panE gene is expressed in the microorganisms by plasmid vectors.

4. Process according to claims 1 and 2, **characterised in that** in order to achieve over-expression the lac-UV5 mutation of the lac promoter or the tac promoter is used as promoter.

5. Process according to claim 1, **characterised in that** microorganisms are used in which the avtA gene is eliminated.

6. Process according to claim 1, **characterised in that** microorganisms are used in which the ilvE gene is eliminated.

7. Process according to claim 1, **characterised in that** microorganisms are used in which in addition the ilvC gene is overexpressed.

8. Process according to claim 1, **characterised in that** microorganisms of the genera Corynebacterium and E. coli are used, in which in addition the ilvC gene from Corynebacterium glutamicum is overexpressed.

9. Process according to claim 1, **characterised in that** in addition one or more of the genes selected from the following group is/are overexpressed:
9.1 the panB gene coding for ketopantoate hydroxymethyltransferase,
9.2 the panD gene coding for aspartate decarboxylase,
9.3 the panC gene coding for pantothenate synthetase.

10. Process according to claim 1, **characterised in that** microorganisms selected from the following group are used:
10.1 Gram-negative bacteria,
10.2 Gram-positive bacteria,
10.3 fungi,
10.4 yeasts.

11. Process according to claim 10, **characterised in that** the Gram-negative microorganisms are Enterobacteriaceae.

12. Process according to claim 11, **characterised in that** the microorganisms are the species Escherichia coli.

13. Process according to claim 10, **characterised in that** the Gram-positive microorganisms are microorganisms of the genus Corynebacterium.

14. Process according to claim 1, **characterised in that** microorganisms are used that have a resistance to L-valine.

15. Process according to claim 11, **characterised in that** the strain FE7 is used that carries an avtA::aadB mutation in the chromosome.

16. Process according to claim 10 or claim 12, **characterised in that** the strains Saccharomyces cerevisiae JD242 or E. coli FE5 are used, in which the intracellular activity of the enzyme ketopantoate reductase is enhanced by overexpression of the reading frame YHR063c from the species Saccharomyces cerevisiae.

17. Plasmid vector pFE91 that carries the ilvC gene of Corynebacterium glutamicum, **characterised by** the restriction map reproduced in Figure 7.

18. Plasmid vector pJD-YHR063c that contains the YHR063c reading frame of Saccharomyces cerevisiae, **characterised by** the restriction map reproduced in Figure 9.

19. Plasmid vector pFE80, **characterised by** the restriction map reproduced in Figure 6 and deposited in E. coli K12 strain MG 1655/pFE80 under the reference DSM 12414.

20. Plasmid vector pFE65, **characterised by** the restriction map reproduced in Figure 5 and deposited in E. coli K12 strain MG 1655/pFE65 under the reference DSM 12382.

21. Plasmid vector pFE32, **characterised by** the restriction map reproduced in Figure 4 and deposited in E. coli K12 strain MG 1655/pFE32 under the reference DSM 12413.

22. E. coli K12 strain FE6, that carries a valine resistance mutation, deposited under the reference DSM 12379.

23. E. coli K12 strain FE7, in which the avtA gene is replaced by an avtA::aadB fragment, deposited under the reference DSM 12380.

24. Pantothenic acid-producing microorganisms (host cell) of the genera E. coli or Corynebacterium that contain one of the plasmid vectors according to claims 19 to 21 and are optionally resistant to one or more metabolites and/or antimetabolites.

25. Process for the production of pantothenic acid, **characterised in that** the following steps are carried out:
a) fermentation of microorganisms selected from the group comprising Gram-negative bacteria, Gram-positive bacteria, fungi and yeasts, in which the nucleotide sequences (pan E gene) coding for the enzyme ketopantoate reductase are overexpressed,
b) enrichment of the pantothenic acid in the medium or in the cells of the microorganisms,
c) isolation of the pantothenic acid.

26. Process according to claim 25, **characterised in that** the Gram-negative bacteria are Enterobacteriaceae.

27. Process according to claim 26, **characterised in that** the bacteria are bacteria of the genus Escherichia.

28. Process according to claim 25, **characterised in that** the Gram-positive bacteria are microorganisms of the genus Corynebacterium.

29. Process according to claim 28, **characterised in that** the bacteria of the genus Corynebacterium are the species Corynebacterium glutamicum.

30. Process according to claim 25, **characterised in that** ketopantoic acid or β-alanine is added in stage a).

## Revendications

1. Procédé pour la préparation d'acide D-pantothénique par fermentation des micro-organismes produisant cet acide,
**caractérisé en ce qu'**
on utilise des micro-organismes dans lesquels on sur-exprime les séquences de nucléotides (gène panE) codant pour la cétopantoate-réductase.

2. Procédé selon la revendication 1,
**caractérisé en ce qu'**
on sur-exprime les séquences de nucléotides codant (gène panE) pour la cétopantoate-réductase à partir de E. coli.

3. Procédé selon la revendication 2,
**caractérisé en ce qu'**
on exprime le gène panE dans les micro-organismes par des vecteurs plasmides.

4. Procédé selon les revendications 1 et 2,
**caractérisé en ce qu'**
on utilise comme promoteur pour obtenir la sur-expression la mutation lac-UV5 du promoteur lac ou le promoteur tac.

5. Procédé selon la revendication 1,
**caractérisé en ce qu'**
on utilise des micro-organismes dans lesquels on inhibe le gène avtA.

6. Procédé selon la revendication 1,
**caractérisé en ce qu'**
on utilise des micro-organismes dans lesquels on inhibe le gène ilvE.

7. Procédé selon la revendication 1,
**caractérisé en ce qu'**
on utilise des micro-organismes dans lesquels on sur-exprime en plus le gène ilvC.

8. Procédé selon la revendication 1,
**caractérisé en ce qu'**
on utilise des micro-organismes des genres Corynebacterium et E.coli, dans lesquels on sur-exprime en plus le gène ilvC de Corynebacterium glutamicum.

9. Procédé selon la revendication 1,
**caractérisé en ce qu'**
on sur-exprime en plus un ou plusieurs des gènes choisis dans le groupe formé par :
9.1. le gène panB codant pour la cétopantoate-hydroxyméthyl-transférase,
9.2. le gène panD codant pour l'aspartate-décarboxylase,
9.3. le gène panC codant pour la pantothénate-synthétase.

10. Procédé selon la revendication 1,
**caractérisé en ce qu'**
on utilise des micro-organismes, choisis dans le groupe :
10.1. des bactéries gram-négatives,
10.2. des bactéries gram-positives,
10.3. des champignons,
10.4. des levures.

11. Procédé selon la revendication 10,
**caractérisé en ce que**
pour les micro-organismes gram-négatifs, il s'agit d'Enterobactériaceae.

12. Procédé selon la revendication 11,
**caractérisé en ce que**
pour les micro-organismes, il s'agit de l'espèce Escherichia coli.

13. Procédé selon la revendication 10,
**caractérisé en ce que**
pour les micro-organismes gram-positifs, il s'agit de micro-organismes du genre Corynebacterium.

14. Procédé selon la revendication 1,
**caractérisé en ce qu'**
on utilise des micro-organismes qui portent une résistance à la L-valine.

15. Procédé selon la revendication 11,
**caractérisé en ce qu'**
on utilise la souche FE7, qui porte une mutation avtA::aadB dans le chromosome.

16. Procédé selon la revendication 10 ou 12,
**caractérisé en ce qu'**
on utilise les souches Saccaromyces cerevisiae JD 1242 ou E. coli FE5, dans lesquelles on élève l'activité intracellulaire de l'enzyme cétopantoate-réductase par sur-expression de la grille de lecture YHR063c de l'espèce Saccharomyces cerevisiae.

17. Vecteur plasmidique pife91, qui porte le gène ilvC de Corynebacterium glutamicum,
**caractérisé par**
la carte de restriction reproduite figure 7.

18. Vecteur plasmidique pJD-YHR063c, qui contient la grille de lecture YHR063c de Saccaromyces cerevisiae,
**caractérisé par**
la carte de restriction reproduite figure 9.

19. Vecteur plasmidique pFE80,
**caractérisé par**
la carte de restriction reproduite sur la figure 6 et déposé dans E. coli K12 souche MG 1655/pFE80 sous la dénomination DSM 12414.

20. Vecteur plasmidique pFE65,
**caractérisé par**
la carte de restriction reproduite figure 5 et déposé dans E. coli K12 souche MG1655/pFE65 sous la dénomination DSM 12382.

21. Vecteur plasmidique pFE32,
**caractérisé par**
la carte de restriction reproduite figure 4 et déposé dans E. coli K12 souche MG1655/pFE32 sous la dénomination DSM 12413.

22. E. coli K12 souche FE6, qui porte une mutation de résistance à la valine, déposé sous la dénomination DSM 12379.

23. E. coli K12 souche FE7, dans lequel le gène avtA est échangé contre un fragment avtA::aadB, déposé sous la dénomination DSM 12380.

24. Micro-organismes produisant de l'acide pantothénique (cellule hôte) des genres E. coli ou Corynebacterium, qui renferment l'un des vecteurs plasmidiques selon les revendications 19 à 21, et le cas échéant, une ou plusieurs résistance(s) métabolite(s) et/ou antimétabolite(s).

25. Procédé pour la préparation de l'acide pantothénique,
**caractérisé en ce qu'**
on met en oeuvre les étapes suivantes :
a) fermentation de micro-organismes, choisis dans le groupe des bactéries gram-négatives, des bactéries gram-positives, des champignons et des levures, dans lequel on surexprime les séquences de nucléotides (gène panE) codant pour l'enzyme cétopantoate-réductase,
b) enrichissement de l'acide pantothénique dans le milieu ou dans les cellules des micro-organismes,
c) isolation de l'acide pantothénique.

26. Procédé selon la revendication 25,
**caractérisé en ce que**
pour les bactéries gram-négatives, il s'agit d'Enterobacteriaceae.

27. Procédé selon la revendication 26,
**caractérisé en ce qu'**
il s'agit de bactéries du genre Escherichia.

28. Procédé selon la revendication 25,
**caractérisé en ce que**
pour les bactéries gram-positives, il s'agit de micro-organismes du genre Corynebacterium.

29. Procédé selon la revendication 28,
**caractérisé en ce que**
pour les bactéries du genre Corynebacterium, il s'agit de l'espèce Corynebacterium glutamicum.

30. Procédé selon la revendication 25,
**caractérisé en ce que**
à l'étape a) on ajoute de l'acide cétopantoïque ou de la β-alanine.
